# EUROPEAN PATENT SPECIFICATION

(11) **EP 2 029 168 B1**
(45) Date of publication and mention of the grant of the patent: **03.10.2012**
(21) Application number: 07798067.0
(22) Date of filing: 04.06.2007
(51) Int. Cl.: A61K 39/21, A61K 39/12, A61K 39/38, C07K 14/16

(54) **HIV-1 CLADE A CONSENSUS SEQUENCES, ANTIGENS, AND TRANSGENES**
HIV-1-CLADE-A-KONSENSSEQUENZEN, -ANTIGENE UND -TRANSGENE
SÉQUENCES CONSENSUS, ANTIGÈNES ET TRANSGÈNES DU VIH-1 DE CLADE A

(30) Priority: 02.06.2006 US 810816 P
(43) Date of publication of application: 04.03.2009
(60) Divisional application: 11005764.3
(73) Proprietor: International AIDS Vaccine Initiative, New York, NY 10038-3901 (US)
(72) Inventor: GUPTA, Kalpana, New York, NY 10022 (US); JACKSON, Nicholas, London SW18 3QR (GB)
(74) Representative: Schüssler, Andrea
(86) International application number: PCT/US2007/070321
(87) International publication number: WO 2007/143606

(56) References cited:
- WO-A2-01/47955
- WO-A2-2005/047483
- MALM MARIA ET AL: "CROSS-CLADE PROTECTION INDUCED BY HUMAN IMMUNODEFICIENCY VIRUS-1 DNA IMMUNOGENS EXPRESSING CONSENSUS SEQUENCES OF MULTIPLE GENES AND EPITOPES FROM SUBTYPES A, B, C, AND FGH" VIRAL IMMUNOLOGY, MARY ANN LIEBERT, INC., NEW YORK, US LNKD- DOI:10.1089/VIM.2005.18.678, vol. 18, no. 4, 1 January 2005 (2005-01-01), pages 678-688, XP009081289 ISSN: 0882-8245
- HOWARD T M ET AL: "Genomic structure and nucleotide sequence analysis of a new HIV type 1 subtype A strain from Nigeria." AIDS RESEARCH AND HUMAN RETROVIRUSES 10 OCT 1996 LNKD- PUBMED:8893049, vol. 12, no. 15, 10 October 1996 (1996-10-10), pages 1413-1425, XP002599105 ISSN: 0889-2229
- 'HIV-1 Sequence Alignment of Clade A env gene' LOS ALAMOS NATIONAL LABORATORY WEB SITE 1997, pages 1 - 2
- GAO ET AL.: 'Molecular cloning and analysis of functional envelope genes from human immunodeficiency virus type 1 sequence subtypes A through G' THE WHO AND NIAID NETWORKS FOR HIV ISOLATION AND CHARACTERIZATION vol. 70, no. 3, March 1996, pages 1651 - 1667, XP002123321

## Description

### FIELD OF THE INVENTION

The invention provides a polynucleotide encoding an HIV-1 Clade A fusion protein comprising HIV-1 Clade A Gag, Pol and Nef antigens as defined in claim 1, an immunogenic composition comprising this polynucleotide as well as this polynucleotide for use in a method of generating an immune response against HIV-1.

The present disclosure relates to consensus nucleotide and protein sequences for HIV-1 Clade A antigens, and to nucleotide and protein sequences for Clade A antigens from circulating HIV-1 field isolates wherein the antigen sequences are closely related to the these consensus sequences. In a preferred embodiment, the present disclosure relates to HIV-1 Clade A transgenes that are derived from such sequences, and that encode HIV-1 Clade A Gag, Pol (RT and Int), and Nef (referred to as "GRIN"). The application also relates to vectors containing such transgenes, including in a preferred embodiment, adenovirus vectors containing such transgenes. The disclosure also relates to immunogenic compositions comprising the HIV-1 Clade A antigens, nucleotide sequences, vectors, or transgenes, and to methods of generating an immune response against HIV-1 in a subject by administering an effective amount of such immunogenic compositions.

### BACKGROUND OF THE INVENTION

AIDS, or Acquired Immunodeficiency Syndrome, is caused by human immunodeficiency virus (HIV) and is characterized by several clinical features including wasting syndromes, central nervous system degeneration and profound immunosuppression that results in opportunistic infections and malignancies. HIV is a member of the lentivirus family of animal retroviruses, which include the visna virus of sheep and the bovine, feline, and simian immunodeficiency viruses (SIV). Two closely related types of HIV, designated HIV-1 and HIV-2, have been identified thus far, of which HIV-1 is by far the most common cause of AIDS. However, HIV-2, which differs in genomic structure and antigenicity, causes a similar clinical syndrome.

An infectious HIV particle consists of two identical strands of RNA, each approximately 9.2 kb long, packaged within a core of viral proteins. This core structure is surrounded by a phospholipid bilayer envelope derived from the host cell membrane that also includes virally-encoded membrane proteins (Abbas et al., Cellular and Molecular Immunology, 4th edition, W.B. Saunders Company, 2000, p. 454). The HIV genome has the characteristic 5'-LTR-Gag-Pol-Env-LTR-3' organization of the retrovirus family. Long terminal repeats (LTRs) at each end of the viral genome serve as binding sites for transcriptional regulatory proteins from the host and regulate viral integration into the host genome, viral gene expression, and viral replication.

The HIV genome encodes several structural proteins. The Gag gene encodes core structural proteins of the nucleocapsid core and matrix. The Pol gene encodes reverse transcriptase (RT), integrase (Int), and viral protease enzymes required for viral replication. The tat gene encodes a protein that is required for elongation of viral transcripts. The rev gene encodes a protein that promotes the nuclear export of incompletely spliced or unspliced viral RNAs. The Vif gene product enhances the infectivity of viral particles. The vpr gene product promotes the nuclear import of viral DNA and regulates G2 cell cycle arrest. The vpu and nef genes encode proteins that down regulate host cell CD4 expression and enhance release of virus from infected cells. The Env gene encodes the viral envelope glycoprotein that is translated as a 160-kilodalton (kDa) precursor (gp160) and cleaved by a cellular protease to yield the external 120-kDa envelope glycoprotein (gp120) and the transmembrane 41-kDa envelope glycoprotein (gp41), which are required for the infection of cells (Abbas, pp. 454-456). Gp140 is a modified form of the env glycoprotein which contains the external 120-kDa envelope glycoprotein portion and a part of the gp41 portion of env and has characteristics of both gp120 and gp41. The Nef gene is conserved among primate lentiviruses and is one of the first viral genes that is transcribed following infection. *In vitro,* several functions have been described, including down regulation of CD4 and MHC class I surface expression, altered T-cell signaling and activation, and enhanced viral infectivity.

HIV infection initiates with gp120 on the viral particle binding to the CD4 and chemokine receptor molecules (e.g., CXCR4, CCR5) on the cell membrane of target cells such as CD4+ T-cells, macrophages and dendritic cells. The bound virus fuses with the target cell and reverse transcribes the RNA genome. The resulting viral DNA integrates into the cellular genome, where it directs the production of new viral RNA, and thereby viral proteins and new virions. These virions bud from the infected cell membrane and establish productive infections in other cells. This process also kills the originally infected cell. HIV can also kill cells indirectly because the CD4 receptor on uninfected T-cells has a strong affinity for gp120 expressed on the surface of infected cells. In this case, the uninfected cells bind, via the CD4 receptor-gp120 interaction, to infected cells and fuse to form a syncytium, which cannot survive. Destruction of CD4+ T-lymphocytes, which are critical to immune defense, is a major cause of the progressive immune dysfunction that is the hallmark of AIDS disease progression. The loss of CD4+ T cells seriously impairs the body's ability to fight most invaders, but it has a particularly severe impact on the defenses against viruses, fungi, parasites and certain bacteria, including mycobacteria.

The different isolates of HIV-1 have been classified into three groups: M (main), O (outlier) and N (non-M, non-O). The HIV-1 M group dominates the global HIV pandemic (Gaschen et al., (2002) Science 296: 2354-2360). Since the HIV-1 M group began its expansion in humans roughly 70 years ago (Korber et al., Retroviral Immunology, Pantaleo et al., eds., Humana Press, Totowa, NJ, 2001, pp. 1-31), it has diversified rapidly (Jung et al., (2002) Nature 418: 144). The HIV-1 M group consists of a number of different clades (also known as subtypes) as well as variants resulting from the combination of two or more clades, known as circulating recombinant forms (CRFs). Subtypes are defined as having genomes that are at least 25% unique (AIDS epidemic update, December 2002). Eleven clades have been identified and a letter designates each subtype. When clades combine with each other and are successfully established in the environment, as can occur when an individual is infected with two different HIV subtypes, the resulting virus is known as a CRF. Thus far, roughly 13 CRFs have been identified. HIV-1 clades also exhibit geographical preference. For example, Clade A, the second-most prevalent clade, is prevalent in East Africa, while Clade B is common in Europe, the Americas and Australia. Clade C, the most common subtype, is widespread in southern Africa, India and Ethiopia (AIDS epidemic update, December 2002). Even within Clades there is variability in the virus beween different strains and viral isolates.

This genetic variability of HIV creates a scientific challenge to vaccine development. One approach that has been suggested is to develop consensus sequences based on the sequences of multiple different HIV strains, and to develop vaccines based on these consensus sequences. The rationale behind such approaches is that the consenus sequences will encode antigens that are conserved among different HIV strains and that such antigens are therefore likely to be useful in generating immune responses against multiple different strains of HIV. HIV-1 clade A consensus sequences have been generated by others. See for example, Nkolola et al. (2004) Gene Ther. 2004. Jul. 11 (13): 1068-80, and Korber B (eds) et al. Human Retroviruses and AIDS: A Compilation and Analysis of Nucleic Acid and Amino Acid Sequences. Los Alamos National Laboratory: Los Alamos, New Mexico, USA, (1997) which involve transgene RENTA and HIVA derived from consensus clade A sequences. However, the consensus sequences described in these articles appear to have been derived from the HIV-1 clade A consensus sequence obtained from the Los Alamos laboratory, and were not generated in the same way as the consensus sequences of the present invention. In addition, these references do not teach use of sequences from actual recently circulating HIV strains which closely match the consensus sequence. Instead they involve using the consensus sequences themselves.

Malm, M. et al., Viral Immunol. 18:678-688, 1. January 2005 disclose the cross-clade protection induced by HIV-1 DNA immunogens expressing consensus sequences of multiple genes and epitopes from subtypes A, B, C, and FGH. The authors have constructed a vaccine platform with an HIV-1 subtype B DNA immunogen expressing full length consensus sequences from HIV-1 rev, nef, tat, and gag with additional cellular epitope clusters from the env and pol regions, wherein this platform has been extended to three additional plasmids expressing the same immunogens but originating from subtypes A or C or FGH ancestral sequences. The immunogenicity in Balb/c mice revealed strong IFN-γ production in response to *in vitro* re-stimulation with a H-2^{d} restricted gag peptide or even stronger toward an *env* epitope, wherein weak humoral immunity was detected. Gene gun immunization with a cocktail of all four plasmids induced pre-challenge immunity and subsequently a robust frequency of protection after experimental challenge with subtype A or B HIV-1/Murine Leukemia Virus. Thus, the cross-clade protection observed in this challenge experiment demonstrated that these multigene/multiepitope HIV DNA immunogens are likely to be potent immunogens also against the HIV-infection.

WO 2005/047483 A2 provides artificial fusions designed to elicit an anti-HIV immune response, as well as nucleic acid molecules and expressing vectors encoding those proteins, wherein the artificial fusions comprise domains from various HIV proteins, including RT, Env, Nef and Tat proteins, as well as at least one HIV CTL epitope associated with long-term, non progression to AIDS. RENTA is an artificial fusion in which the HIV domains are from an HIV Clade A consensus sequence and contains additional domains, useful for example, in monitoring expression levels or laboratory animal immune response, wherein such domains are optionally included in the artificial fusions.

WO 01/47955 A2 discloses an immunogen in sterile form suitable for administration to a human subject, the immunogen comprising: at least a portion of the gag protein of HIV, said gag protein being from an HIV clade or having consensus sequence for one or more HIV clades, and comprising at least parts of p17 and p24; and a synthetic polypeptide comprising a plurality of amino acid sequences, each sequence comprising a human CTL epitope of an HIV protein, and wherein a plurality of HIV proteins are represented in the synthetic polypeptide, said CTL epitopes being selected to stimulate an immune response to one or more HIV clades of interest.

### SUMMARY OF THE INVENTION

The present disclosure provides new and improved consensus sequences for HIV-1 Clade A antigens. The consensus sequences are particularly advantageous because they are based on the antigen sequences of a large number of different HIV-1 Clade A strains, and also because they are based on the sequences of antigens from recently isolated HIV-1 Clade A strains. Accordingly, the consensus sequences of the present disclosure have superior biological relevance as compared to previously generated HIV-1 Clade A consensus sequences.

Another major advantage of the present disclosure is that it provides HIV-1 Clade A antigens, and strategies for producing such antigens, that are derived from naturally occurring HIV-1 Clade A strains. These antigens are selected such that they are closely related to, or have a small "protein distance" from, the consensus sequences of the present disclosure. An advantage of using these naturally occurring sequences with the closest match to the consensus sequences, as opposed to the artificially generated consensus sequences, is that less genetic manipulations are needed to generate these sequences and importantly biological relevance is assured.

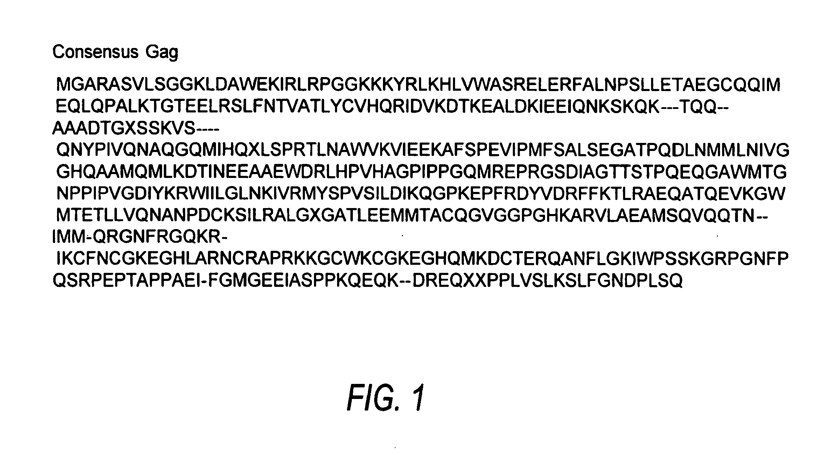

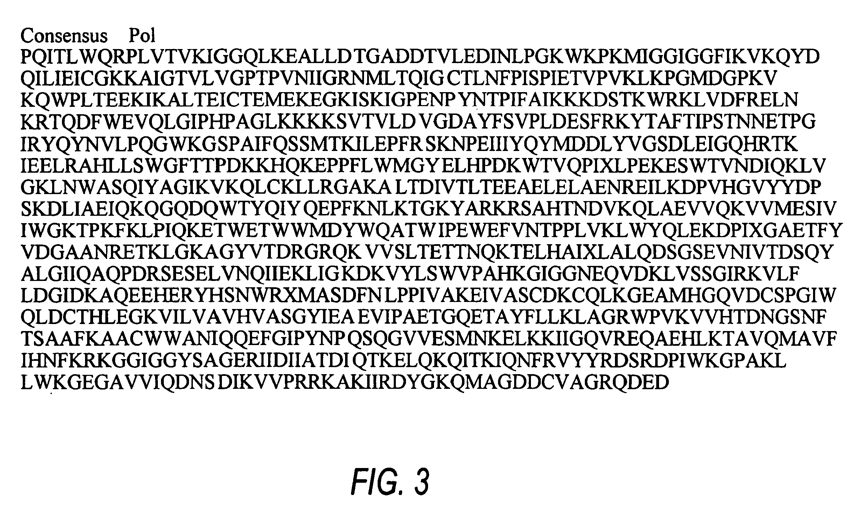

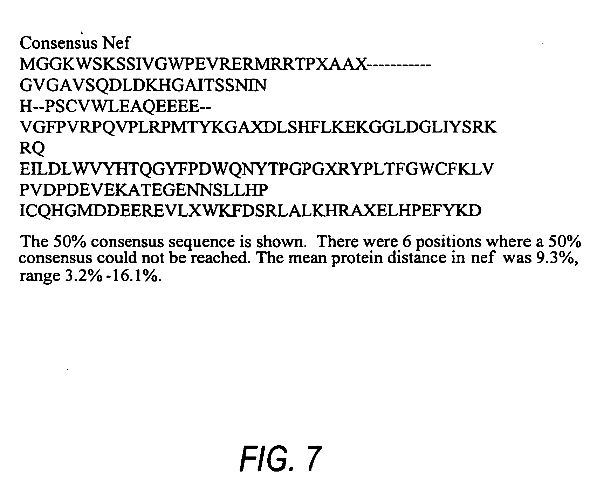

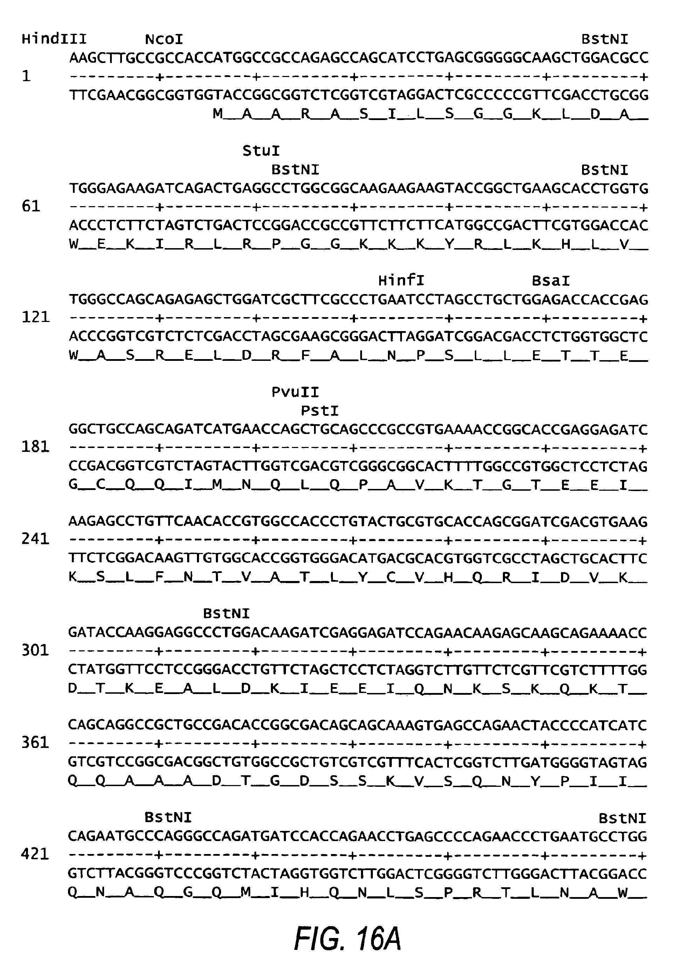

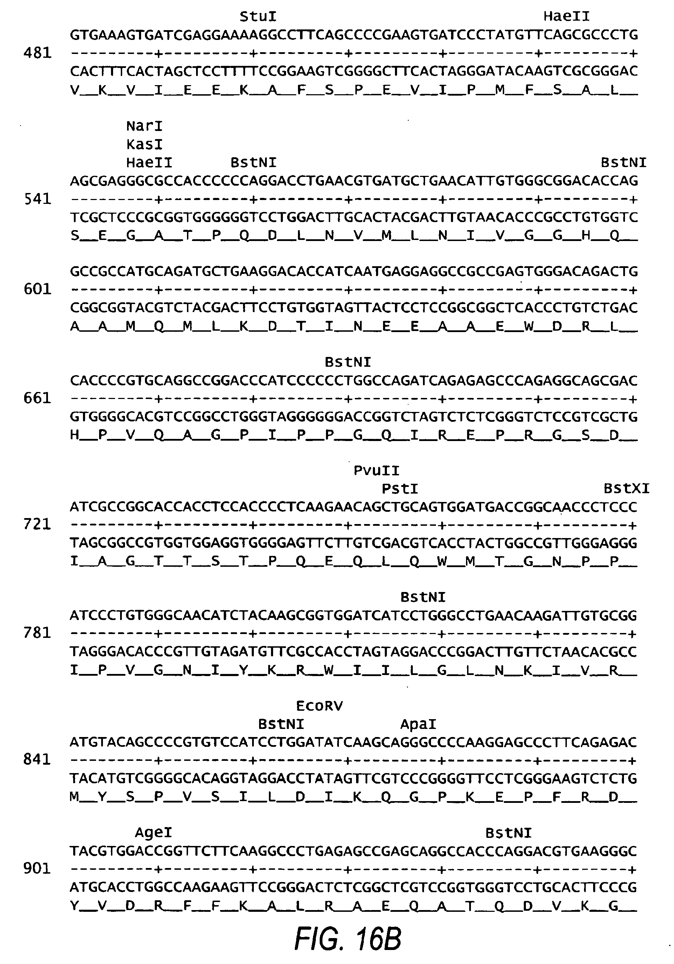

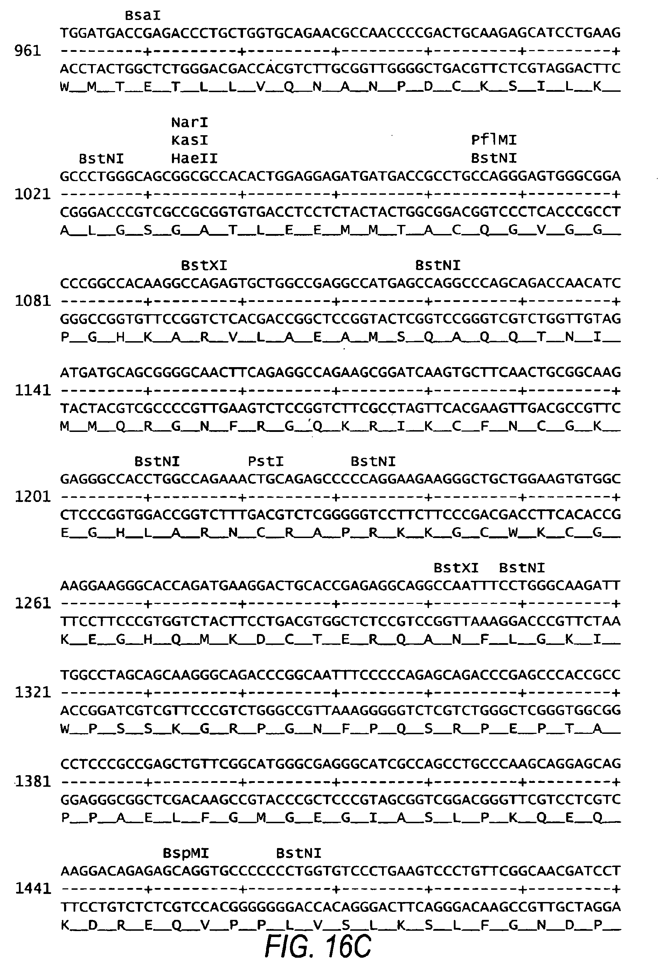

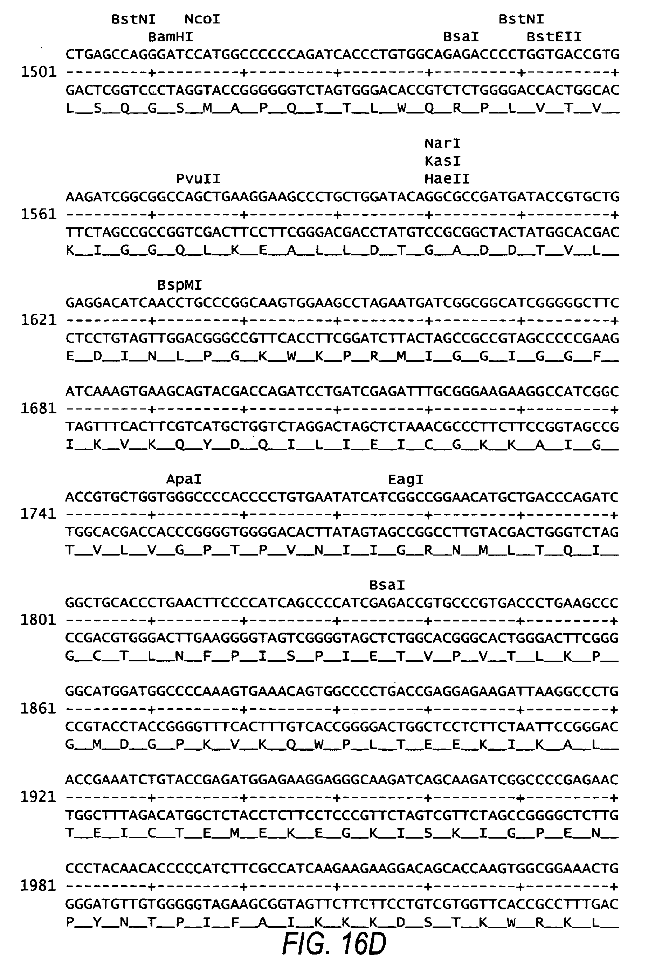

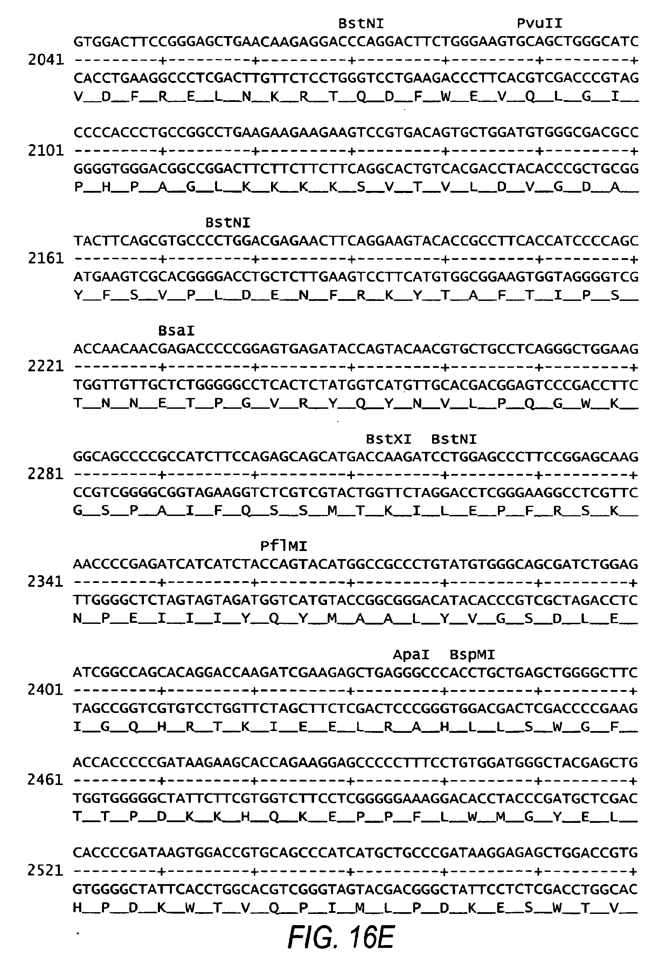

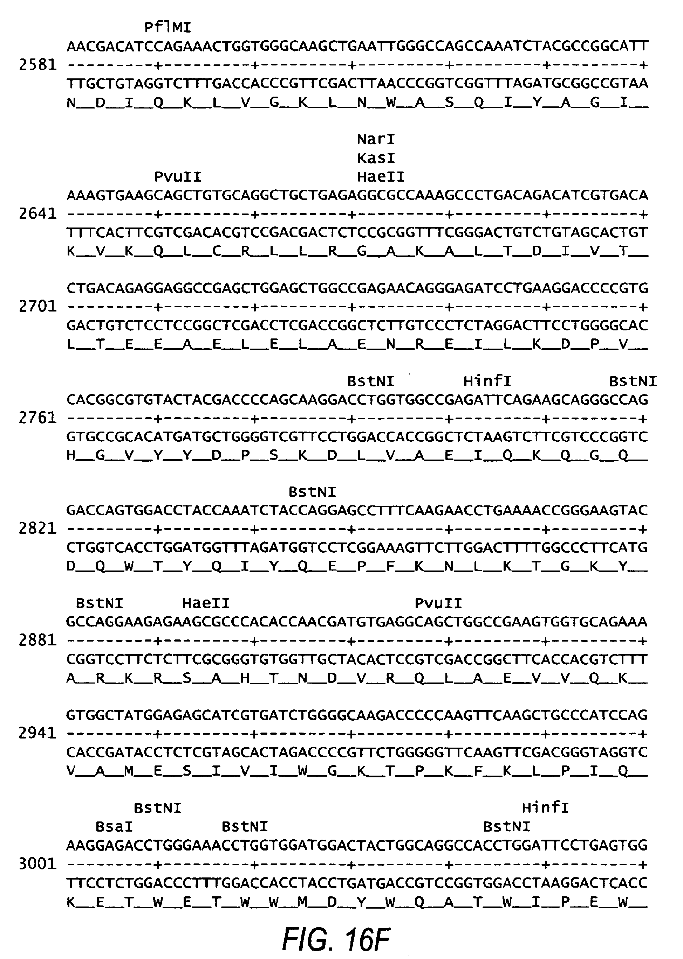

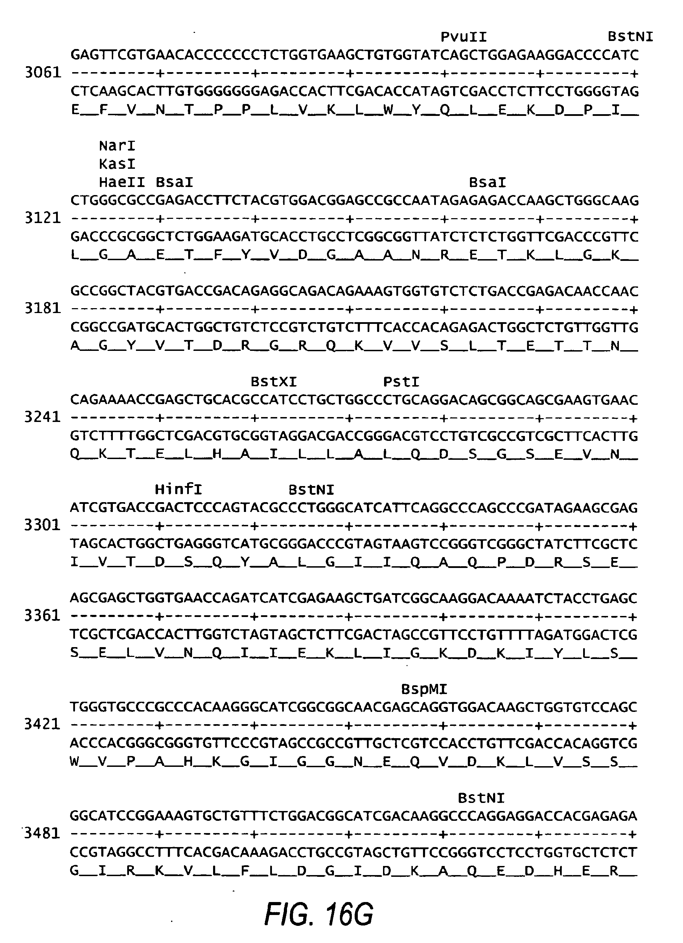

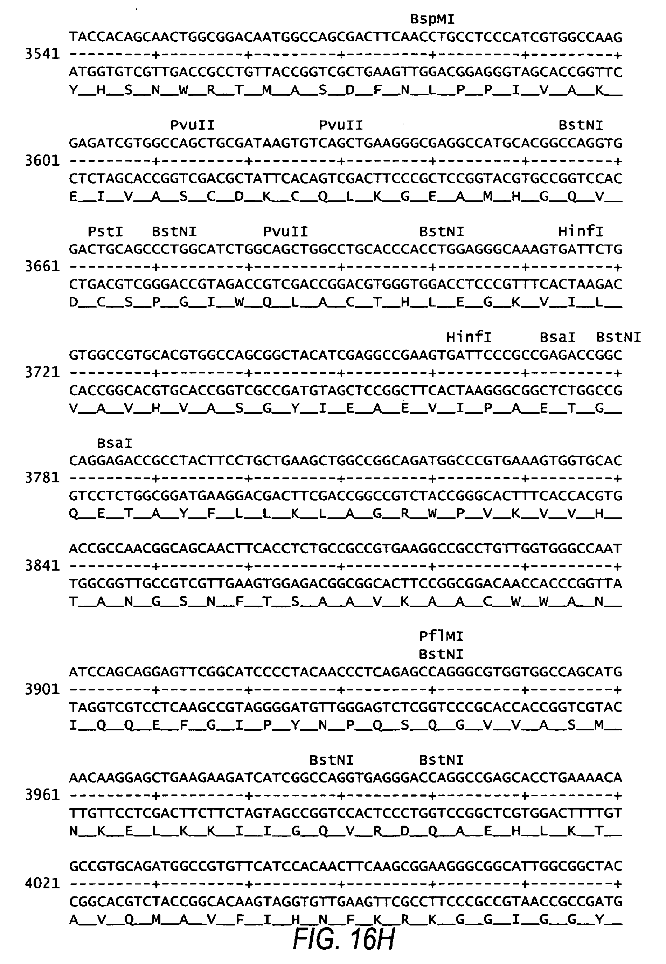

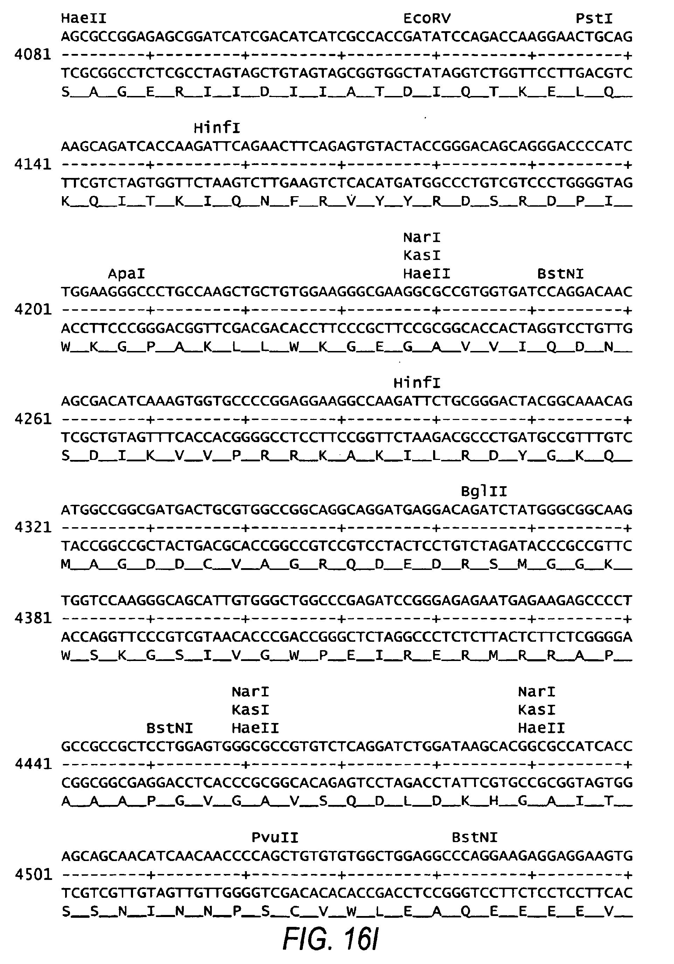

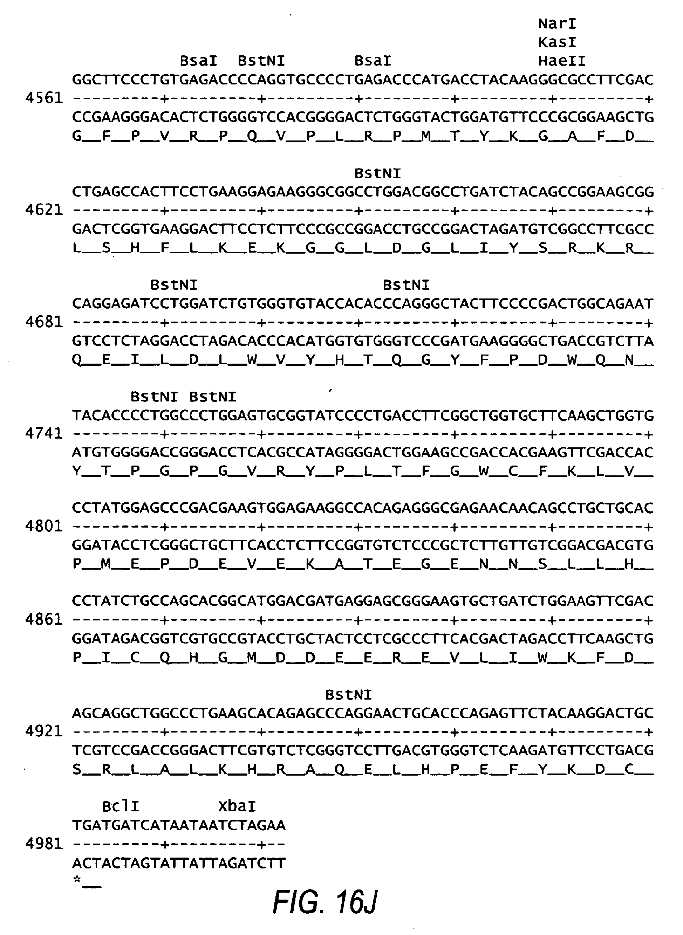

In a first aspec the present invention is directed to a polynucleotide encoding an HIV-1 Clade fusion protein wherein the fusion protein comprises HIV-1 Clade A Gag, Pol and Nef antigens and wherein the fusion protein comprises the amino acid sequence of Figure 16. Particularly, it relates to consensus amino acid sequences for the HIV-1 Clade A antigens Gag, Pol (comprising RT and Int), Nef and Env. In preferred embodiments, the invention relates to the consensus Gag amino acid sequence of FIG. 1, the consensus Pol amino acid sequence of FIG. 3, and/or the consensus Nef amino acid sequence of FIG. 7.

The application is directed to a method of identifying a consensus amino acid sequence for an HIV-1 Clade A antigen of interest comprising determining the amino acid sequence of the antigen of interest in several circulating HIV-1 strains or field isolates, aligning such sequences, and determining the consensus sequence for that antigen.

In addition, the application relates to a method of identifying an HIV-1 Clade A antigen from a circulating strain or field isolate of HIV-1 Clade A that has an amino acid sequence that is similar to the consensus amino acid sequence for that HIV-1 Clade A antigen. In a preferred embodiment the HIV-1 Clade A antigen is selected based the degree of similarity to the consensus sequence, with sequences having the highest degree of similarity to, or the smallest "protein distance" from, the consensus sequence being preferred. In a further preferred embodiment the HIV-1 Clade A antigen is selected from a recently circulating strain or field isolate of HIV-1 Clade A. In a further embodiment the invention relates to HIV 1 Clade A antigens identified using such methods.

In addition, the application relates to a method of identifying an HIV-1 Clade A antigen from a circulating strain or field isolate of HIV-1 Clade A that has an amino acid sequence that is similar to the consensus amino acid sequence for that HIV-1 Clade A antigen, and then making mutations in that sequence to abrogate the biological functions of the sequences. It is preferred that a minimalist approach is used, i.e. that the number of mutations is kept to a minimum so that only those mutations necessary to abrogate function and facilitate obtaining regulatory authority approval are made and un-necessary alteration of the original HIV-1 gene sequences are avoided. For example, in one embodiment the Nef component of GRIN is not altered but rather fusion of the Nef N-terminus to the Int C-terminus abrogates nef function while retaining all the original nucleotide sequences of Nef.

Furthermore, the application relates to a method of improving genetic stability of the HIV-1 Clade A transgene for insertion into viral vector technologies. The PR (protease) component is removed from Gag-full-length Pol-Nef (full length Pol contains PR, and Int and RT) so that only the Int and RT portions of Pol are left. This has the advantage of improved genetic stability and improved cloning and virus rescue properties, particularly using Ad35 and/or Ad11. Removing PR in this way is a minimalist approach in that only the smallest functional subunit of POL is removed, thereby preserving the larger IN & RT functional subunits. The application also relates to HIV-1 Clade A antigens selected and produced using such methods.

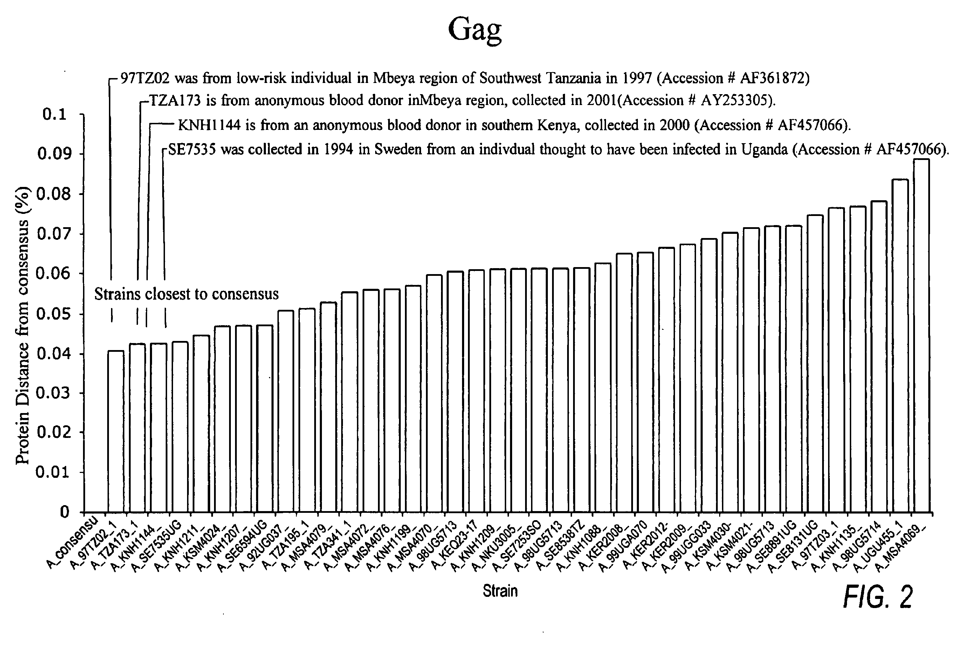

In one embodiment the antigen is a Gag antigen from one of the strains listed in Table 1 and FIG. 2. Preferably the Gag antigen is selected from a strain in which the "protein distance" from the consensus Gag sequence is less than 0.07%, or more preferably less than 0.06%, or more preferably still less than 0.05%. In a preferred embodiment the Gag antigen is from HIV-1 Clade A strain TZA173, strain 97TZ02, strain KNH1144 or strain SE7535UG.

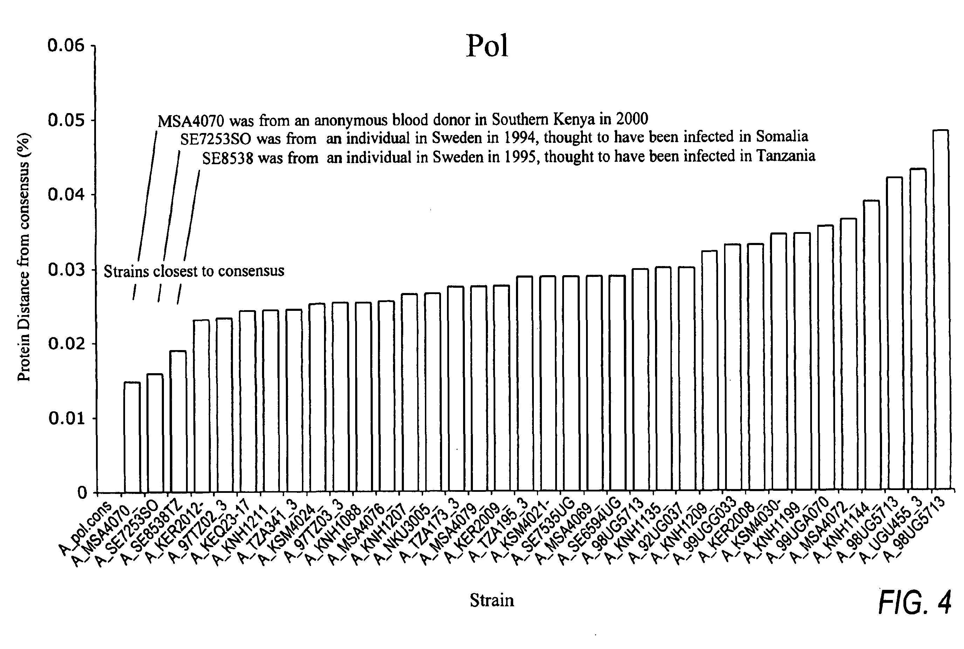

In another embodiment the antigen is a Pol antigen from one of the strains listed in Table 2 and FIG. 4. Preferably the Pol antigen is selected from a strain in which the "protein distance" from the consensus Pol sequence is less than 0.03%, or more preferably less than 0.025%. In a preferred embodiment the Pol antigen is from HIV-1 Clade A strain MSA4070, strain SE7245SO, or strain SE8538.

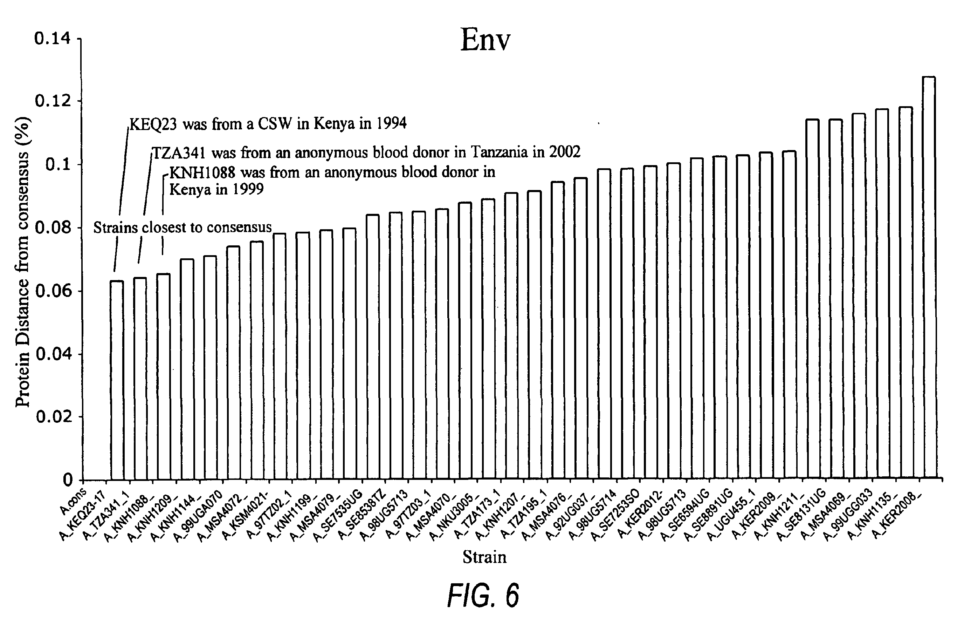

A further antigen is an Env antigen from one of the strains listed in Table 3 and FIG. 6. Preferably the Env antigen is selected from a strain in which the "protein distance" from the consensus Gag sequence is less than 0.1, or more preferably less than 0.08%, or more preferably less than 0.07%, or more preferably still less than 0.065%. In a preferred embodiment the Env antigen is from HIV-1 Clade A strain KEQ23, strain TZA341, or strain KNH1088.

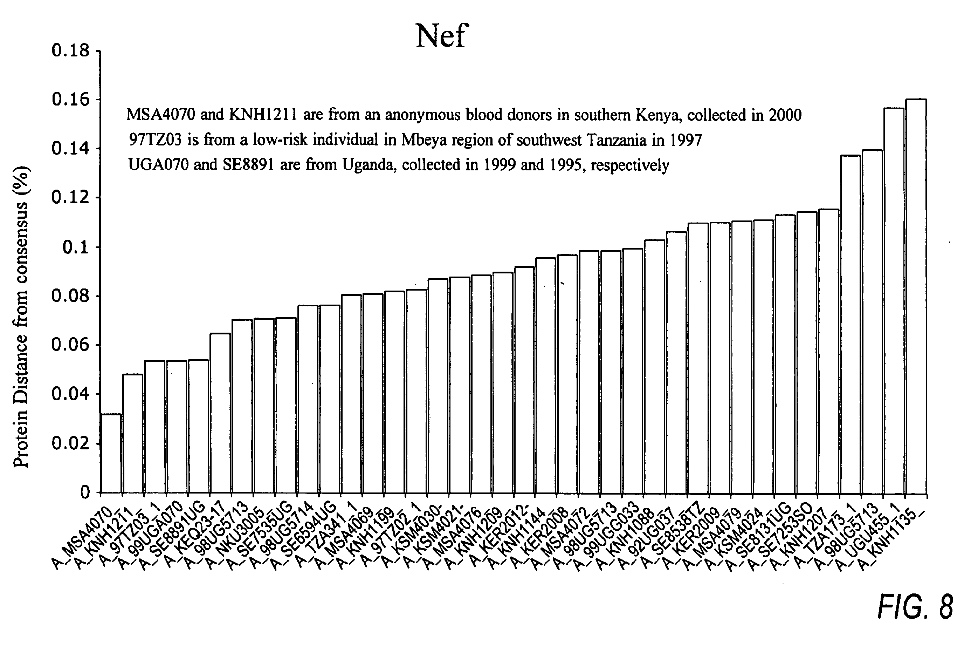

In another embodiment the antigen is a Nef antigen from one of the strains listed in Table 4 and FIG. 8. Preferably the Nef antigen is selected from a strain in which the "protein distance" from the consensus Gag sequence is less than 0.1%, or more preferably less than 0.08%, or more preferably less than 0.07%, or more preferably less than 0.06, or more preferably still, less than 0.05%. In a preferred embodiment the Nef antigen is from HIV-1 Clade A strain MSA4070, or strain KNH1211, or strain 97TZ03, or strain 99UGA070, or strain SE8891 UG.

The present invention is directed to the nucleotide sequences that encode the HIV-1 Clade A antigens as defined in claim 1. The application also relates to vectors comprising these nucleotide sequences. The nucleotide sequences of the invention, and the vectors that comprise them, and also the antigens encoded by the nucleotide sequences of the invention, are useful in generating an immune response against HIV Clade A antigens *in vivo* and are useful in the production of vaccines against HIV-1 Clade A strains. The nucleotide sequences of the invention may also be useful for expressing and producing the HIV-1 Clade A antigens that they encode in cells or in vitro, for example, so that the antigens may be produced, isolated, and/or purified.

The nucleotides of the invention may be altered as compared to the consensus nucleotide sequences, or as compared to the sequences from circulating HIV-1 isolates that are closely related to such consensus sequences. For example, in one embodiment the nucleotide sequences may be mutated such that the activity of the encoded proteins in vivo is abrogated. In another embodiment the nucleotide sequences may be codon optimized, for example the codons may be optimized for human use. Preferably, the nucleotide sequences of the invention are both mutated to abrogate the normal in vivo function of the encoded proteins, and codon optimized for human use. For example, each of the Gag, Pol, Env, Nef, RT, and Int sequences may be altered in these ways.

In a preferred embodiment, a single nucleotide sequence encodes a fusion protein comprising the Gag, RT (part of Pol) and Nef antigens. As used herein the abbreviations "GRN" and "GRtN" are used interchangeably to refer to HIV-1 Clade A fusion proteins comprising the Gag, RT and Nef antigens and to refer to the nucleotide sequences that encode these fusion proteins. In a still more preferred embodiment the nucleotide sequence encoding GRN is inserted into a vector suitable for allowing expression of the GRN fusion protein. Preferably the vector is an adenovirus vector selected from the group consisting of Ad5, Ad35, Ad11, C6, and C7.

In another preferred embodiment a single nucleotide sequence encodes a fusion protein comprising the Gag, Pol (includes RT and Int) and Nef antigens. As used herein the abbreviations "GRIN" and "GRtIN" are used interchangeably to refer to HIV-1 Clade A fusion proteins comprising the Gag, Pol and Nef antigens and to refer to the nucleotide sequences that encode these fusion proteins. In even more preferred embodiments GRIN has the amino acid sequence illustrated in FIGS. 16A-16J and is encoded by the nucleotide sequence illustrated in FIGS. 16A-16J. In a still more preferred embodiment the nucleotide sequence encoding GRIN is inserted into a vector suitable for allowing expression of the GRIN fusion protein. Preferably the vector is an adenovirus vector, more preferably and adenovirus vector selected from the group consisting of Ad5, Ad35, Ad11, C6, and C7.

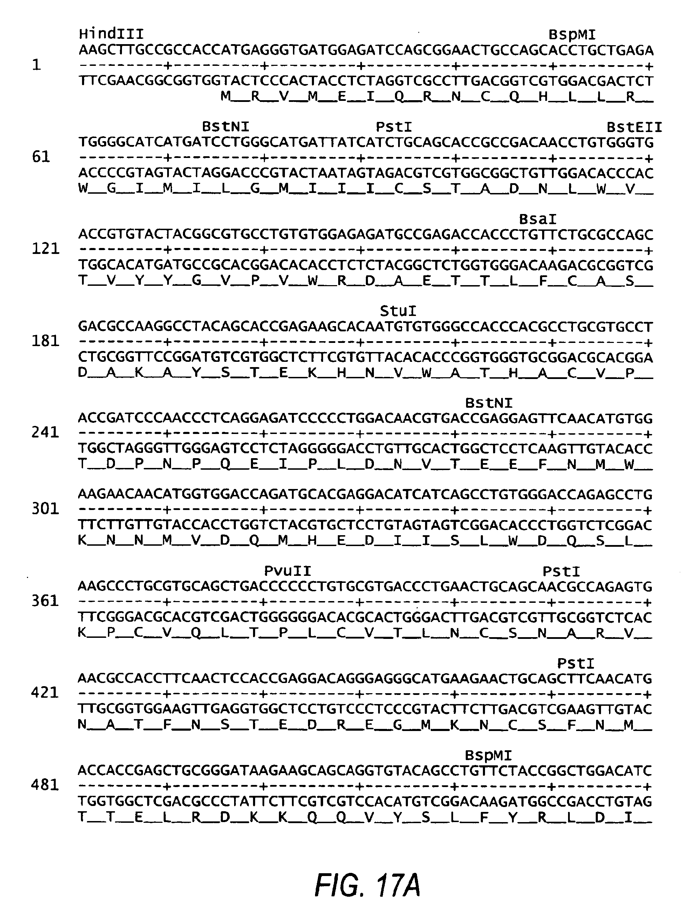

Within the disclosure a single nucleotide sequence of the invention encodes an HIV-1 Clade A Env antigen. The Env antigen has the amino acid sequence illustrated in FIGS. 17A-17D and is encoded by the nucleotide sequence illustrated in FIGS. 17A-17D. The nucleotide sequence encoding Env is inserted into a vector suitable for allowing expression of the Env protein. Preferably the vector is an adenovirus vector, more preferably and adenovirus vector selected from the group consisting of Ad5, Ad35, Ad11, C6, and C7.

The disclosure provides methods of generating an immune response against HIV-1 Clade A antigens comprising administering to a subject a nucleotide sequence or antigen according to the invention. In preferred embodiments the method of generating an immune response against HIV-1 Clade A comprises administering a nucleotide sequence encoding either GRIN or GRN wherein the nucleotoide sequence is contained in an adenovirus vector selected from the group consisting of Ad5, Ad35, Ad11, C6, and C7. In further preferred embodiments, the vectors comprising GRIN or GRN are co-administered with a vector comprising a nucleotide sequence encoding an Env antigen of the invention.

In a further embodiment, the present disclosure provides immunogenic compositions or vaccine compositions comprising the nucleotide sequences of the disclosure. The invention provides an immunogenic composition comprising the polynucleotide of claim 1 as well as the polynucleotide of claim 1 for use in a method of generating an immune response against HIV-1. It should be noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

The following Detailed Description, given by way of example, but not intended to limit the invention to the specific embodiments described, may be best understood in conjunction with the accompanying Figures.
FIG. 1 is a consensus amino acid sequence of the Gag protein of HIV-1 Clade A.
FIG. 2 is a graph illustrating the "distance" of the Gag protein sequences of circulating HIV-Clade A strains to that of the consensus HIV-1 Clade A Gag protein sequence.
FIG. 3 is a consensus amino acid sequence of the Pol protein of HIV-1 Clade A.
FIG. 4 is a graph illustrating the "distance" of the Pol protein sequences of circulating HIV-Clade A strains to that of the consensus HIV-1 Clade A Pol protein sequence.
FIG. 5 is a consensus amino acid sequence of the Env protein of HIV-1 Clade A.
FIG. 6 is a graph illustrating the "distance" of the Env protein sequences of circulating HIV-Clade A strains to that of the consensus HIV-1 Clade A Env protein sequence.
FIG. 7 is a consensus amino acid sequence of the Nef protein of HIV-1 Clade A.
FIG. 8 is a graph illustrating the "distance" of the Nef protein sequences of circulating HIV-Clade A strains to that of the consensus HIV-1 Clade A Nef protein sequence.
FIG. 9 is a schematic representation of the GRIN and GRN transgenes.
FIG. 10 illustrates the amino acid sequence of the Gag protein from HIV-1 Clade A. strain TZA173 having Genbank accession number AY253305.
FIG. 11 illustrates the amino acid sequence of the Pol protein from HIV-1 Clade A strain MSA4070 having Genbank accession number AF457081.
FIG. 12 illustrates the amino acid sequence of the Nef protein from HIV-1 Clade A strain MSA4070 having Genbank accession number AF457081.
FIG. 13 illustrates the amino acid sequence of the Env protein from HIV-1 Clade A strain TZA341 having Genbank accession number AY253314.
FIGS. 14A-14C provide a sequence of GRIN as inserted into the Ad35 vector.
FIGS. 15A-15B provide a sequence of Env as inserted into the Ad35 vector.
FIGS. 16A-16J provide nucleotide and amino acid sequences of the codon optimized GRIN transgene.
FIGS. 17A-17D provide nucleotide and amino acid sequences of the codon optimized Env transgene.

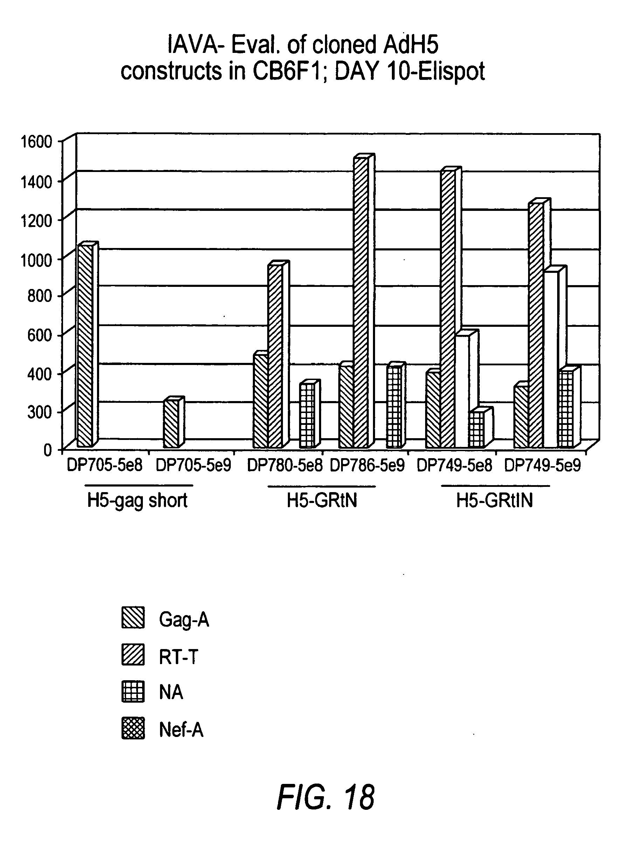

FIG. 18 illustrates graphically the immunogenicity of Ad5-GRIN and Ad5-GRN in mice as measured by IFN-gamma ELIspot assay.

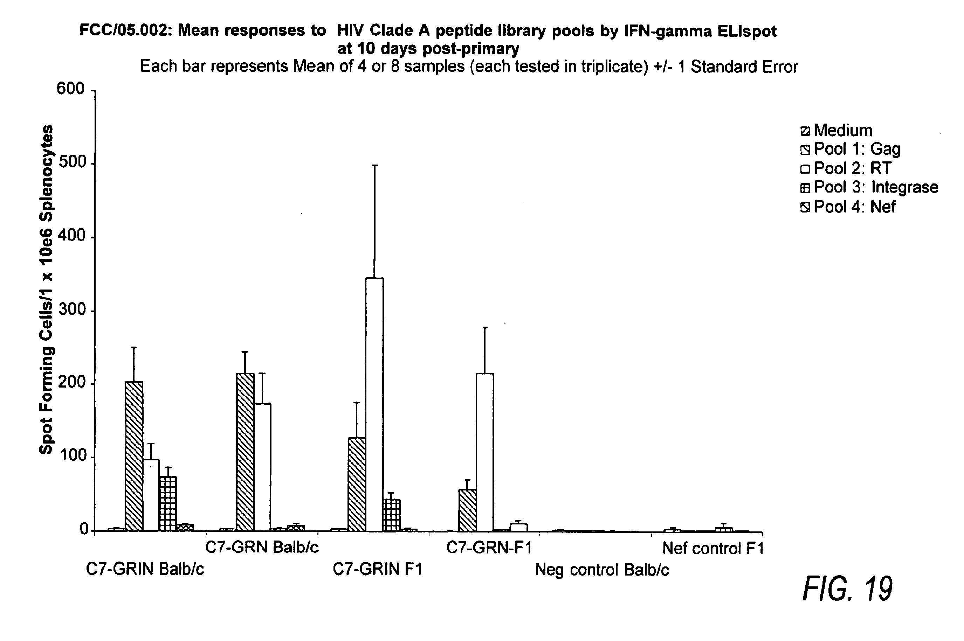

FIG. 19 illustrates graphically the immunogenicity of C7-GRIN and C7-GRN in mice as measured by IFN-gamma ELIspot assay.

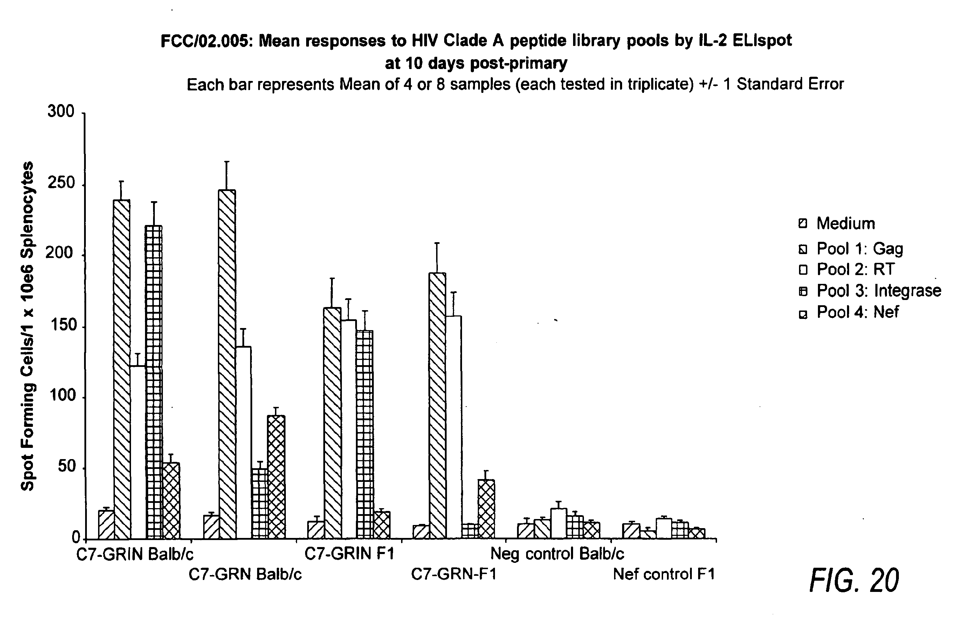

FIG. 20 illustrates graphically the immunogenicity of C7-GRIN and C7-GRN in mice as measured by IL-2 ELIspot assay.

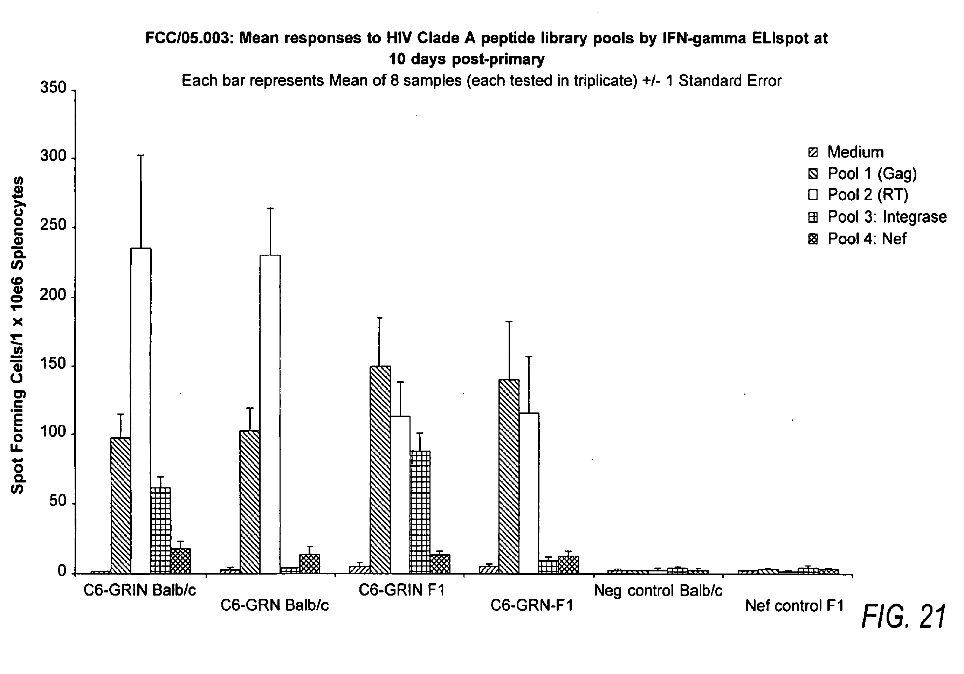

FIG. 21 illustrates graphically the immunogenicity of C6-GRIN and C6-GRN in mice as measured by IFN-gamma ELIspot assay.

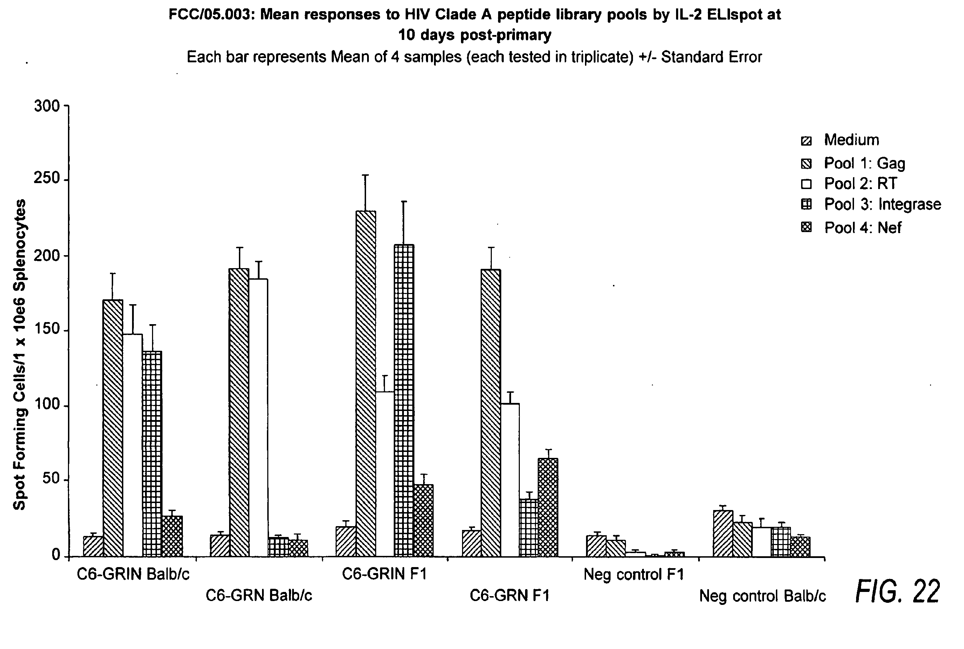

FIG. 22 illustrates graphically the immunogenicity of C6-GRIN and C6-GRN in mice as measured by IL-2 ELIspot assay.

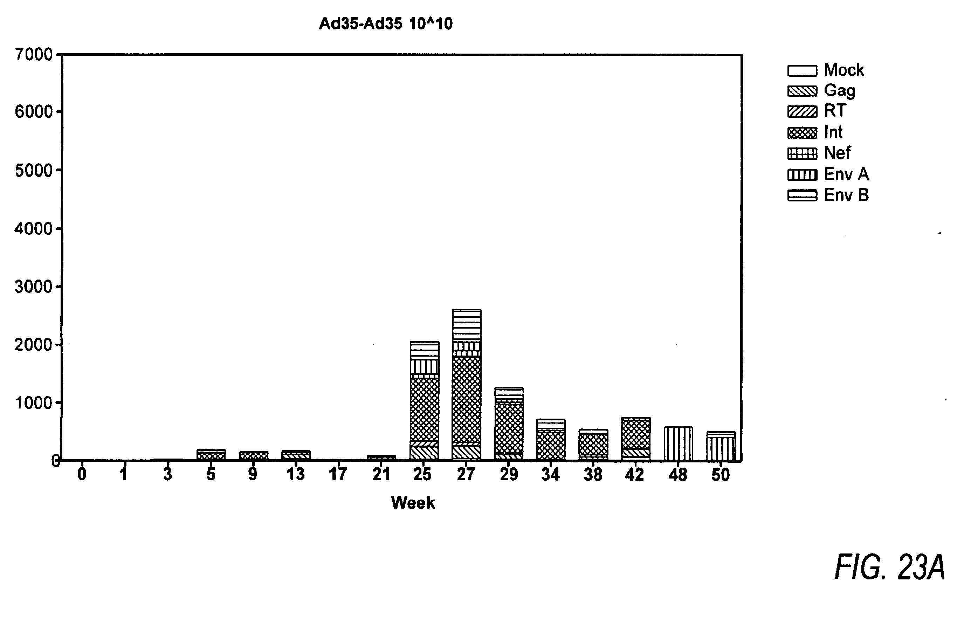

FIG. 23A illustrates IFN-γ ELISpot immunogenicity of Ad35-GRIN/ENV at the 10¹⁰ vp dose following a month 0-6 immunization schedule in rhesus macaques. Definition of Positive Response: For a single peptide pool from a single sample: Response = (mean peptide count - mean no-peptide count). To be positive, a single peptide response must satisfy: 1. Mean peptide count > 4x mean no-peptide count from same plate; 2. Coefficient of variation amongst replicate counts ≤70% &3. Response > 55 SFC/106. Geometric mean responses for Spot Forming Cells (SFC) per million PBMCs to each antigen component (Gag, RT, IN and ENV) are shown on the γ-axis and bleed timepoints in weeks on the x-axis.

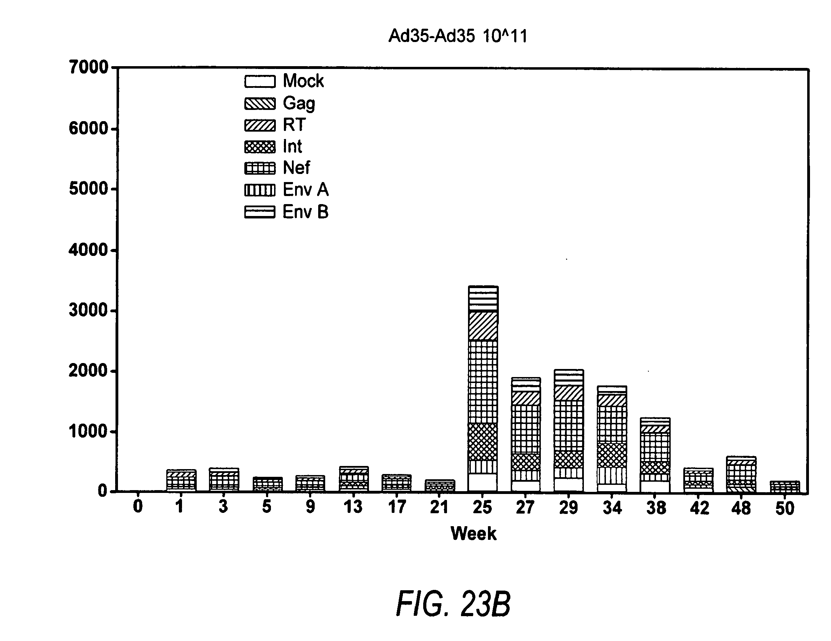

FIG. 23B illustrates IFN-γ ELISpot immunogenicity of Ad35-GRIN/ENV at the 10¹¹ vp dose following a month 0-6 immunization schedule in rhesus macaques. Definition of Positive Response: For a single peptide pool from a single sample: Response = (mean peptide count - mean no-peptide count). To be positive, a single peptide response must satisfy: 1. Mean peptide count > 4x mean no-peptide count from same plate; 2. Coefficient of variation amongst replicate counts ≤70% &3. Response > 55 SFC/106. Geometric mean responses for Spot Forming Cells (SFC) per million PBMCs to each antigen component (Gag, RT, IN and ENV) are shown on the γ-axis and bleed timepoints in weeks on the x-axis.

### DETAILED DESCRIPTION OF THE INVENTION

The present disclosure relates to consensus nucleotide and protein sequences for HIV-1 clade A antigens, and to circulating HIV-1 field isolates that closely match these consensus sequences. The disclosure also relates to altered version of these sequences, which may be altered such that the function of the gene products in vivo is abrogated, to constructs and vectors comprising the sequences of the invention, and to immunogens, immunogenic the compositions, and vaccines made using the sequences of the disclosure. The disclosure also relates to methods of generating an immune response against HIV-1 Clade A antigens in a subject and to methods of inducing protective immunity against challenge with HIV-1. The various embodiments of the invention are summarized above in the section entitled "Summary of the Invention." Further details of the invention are provided in the Detailed Description and Examples that follow, and also in the Drawings.

As described in the above "Summary of the Invention" and the "Examples" below, the present disclosure provides HIV-1 Clade A consensus antigens, and also antigens from circulating HIV-1 Clade A strains that are closely related to these consensus sequences. The disclosure also provides HIV-1 transgenes and antigens encoded by these transgenes. These transgenes comprise sequences encoding the HIV-1 Clade A antigens of the disclosure. For example, in one preferred embodiment the present disclosure provides a GRIN (also referred to as GRtIN) transgene which comprises Gag, Pol (both RT and Int) and Nef antigens of the disclosure. The disclosure also provides a GRN (also referred to as GRtN) transgene which comprises the Gag, RT and Nef antigens of the disclosure.

The invention provides a polynucleotide encoding an HIV-1 Clade A fusion protein comprising HIV-1 Clade A Gag, Pol and Nef antigens, wherein the fusion protein comprises the amino acid sequence of Figure 16, an immunogenic composition comprising this polynucleotide as well as this polynucleotide for use in a method of generating an immune response against HIV-1.

The terms "protein", "peptide", "polypeptide", and "amino acid sequence" are used interchangeably herein to refer to polymers of amino acid residues of any length. The polymer may be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

As used herein, the terms "antigen" or "immunogen" are used interchangeably to refer to a substance, typically a protein, which is capable of inducing an immune response in a subject. The term also refers to proteins that are immunologically active in the sense that once administered to a subject (either directly or by administering to the subject a nucleotide sequence or vector that encodes the protein) is able to evoke an immune response of the humoral and/or cellular type directed against that protein.

It should be understood that the proteins and antigens of the disclosure may differ from the exact sequences illustrated and described herein. Thus, the disclosure contemplates deletions, additions and substitutions to the sequences shown, so long as the sequences function in accordance with the methods of the disclosure. In this regard, particularly preferred substitutions will generally be conservative in nature, i.e., those substitutions that take place within a family of amino acids. For example, amino acids are generally divided into four families: (1) acidic--aspartate and glutamate; (2) basic--lysine, arginine, histidine; (3) non-polar--alanine, valine, leucine, isoleucine, proline, phenylalanine, methionine, tryptophan; and (4) uncharged polar--glycine, asparagine, glutamine, cystine, serine threonine, tyrosine. Phenylalanine, tryptophan, and tyrosine are sometimes classified as aromatic amino acids. It is reasonably predictable that an isolated replacement of leucine with isoleucine or valine, or vice versa; an aspartate with a glutamate or vice versa; a threonine with a serine or vice versa; or a similar conservative replacement of an amino acid with a structurally related amino acid, will not have a major effect on the biological activity. Proteins having substantially the same amino acid sequence as the sequences illustrated and described but possessing minor amino acid substitutions that do not substantially affect the immunogenicity of the protein are, therefore, within the scope of the disclosure.

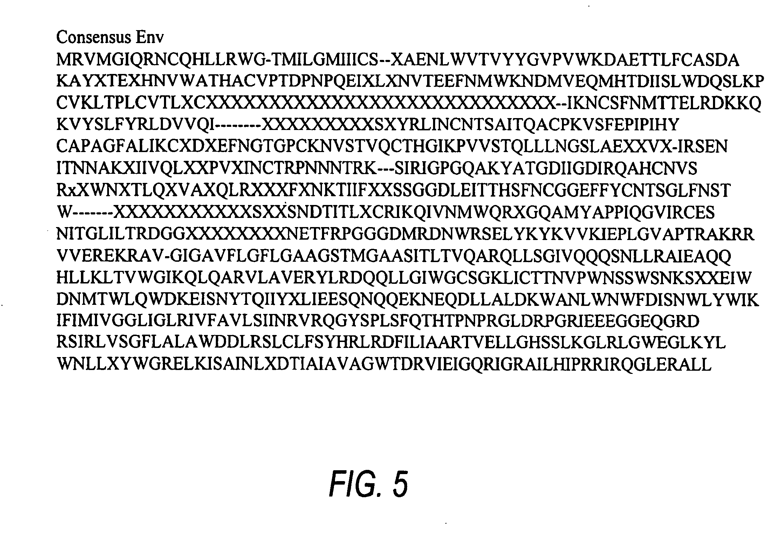

The application describes "consensus" amino acid sequences for an HIV-1 Clade A antigens. It relates to consensus amino acid sequences for the HIV-1 Clade A antigens Gag, Pol (comprising RT and Int), Nef and Env. It relates to a consensus Gag amino acid sequence of FIG. 1, the consensus Pol amino acid sequence of FIG. 3, to a consensus Env amino acid sequence of FIG. 5, and/or a consensus Nef amino acid sequence of FIG. 7. The disclosure is directed to a method of identifying a consensus amino acid sequence for an HIV-1 Clade A antigen of interest comprising obtaining the amino acid sequence of the antigen of interest in several circulating HIV-1 strains or field isolates, aligning such sequences, and determining the consensus sequence for that antigen. For example, in one embodiment a database is generated using available sequences for HIV-1 Clade A non-recombinant circulating strains, and the individual HIV-1 genes (for example gag, pol, nef and env) from all the sequences in the database are then aligned, with dashes inserted to maintain alignment in regions with insertions or deletions in the sequence, and a 50% consensus sequence can then be derived.

The disclosure also relates to methods of identifying antigens from naturally occurring HIV-1 Clade A strains that have an amino acid sequence that has a small "protein distance" from the consensus amino acid sequence of that antigen. The "protein distance" is a measure of the level of similarity or difference between two amino acid sequences. Two amino acid sequences that are very similar have a low protein distance. Two amino acid sequences that are very different have a high protein distance. Protein distances are preferably calculated using the Dayhoff PAM250 substitution matrix (M.O. Dayhoff, ed., 1978, Atlas of Protein Sequence and Structure, Vol. 5) which weights substitutions according to the degree of biochemical similarity. However, other methods for determining protein distance can also be used.

As used herein the terms "nucleotide sequences" and "nucleic acid sequences" refer to deoxyribonucleic acid (DNA) or ribonucleic acid (RNA) sequences, including, without limitation, messenger RNA (mRNA), DNA/RNA hybrids, or synthetic nucleic acids. The nucleic acid can be single-stranded, or partially or completely double-stranded (duplex). Duplex nucleic acids can be homoduplex or heteroduplex.

As described in the above "Summary of the Invention" and the "Examples" below, the present disclosure provides HIV-1 Clade A consensus antigens and to the nucleotide sequences that encode these consensus antigen. The disclosure also relates to antigens from circulating HIV-1 Clade A strains that are closely related to these consensus sequences, and to the nucleotide sequences that encode them. The disclosure also provides HIV-1 Clade A transgenes which comprise sequences encoding the HIV-1 Clade A antigens. As used herein the term "transgene" is used to refer to "recombinant" nucleotide sequences that are derived from either the HIV-1 Clade A consensus nucleotide sequences, or from the nucleotide sequences that encode the antigens from recently circulating HIV-1 Clade A strains that have been identified as being closely matched to these consensus sequences. The term "recombinant" means a nucleotide sequence that has been manipulated "by man" and which does not occur in nature, or is linked to another nucleotide sequence or found in a different arrangement in nature. It is understood that manipulated "by man" means manipulated by some artificial means, including by use of machines, codon optimization, restriction enzymes, etc. For example, the GRIN, GRN, and Env transgenes are provided.

The nucleotides may be altered as compared to the consensus nucleotide sequences, or as compared to the sequences from circulating HIV-1 isolates that are closely related to such consensus sequences. For example, in one embodiment the nucleotide sequences may be mutated such that the activity of the encoded proteins in vivo is abrogated. The nucleotide sequences may be codon optimized, for example the codons may be optimized for human use. Preferably the nucleotide sequences are both mutated to abrogate the normal in vivo function of the encoded proteins, and codon optimized for human use. For example, each of the Gag, Pol, Env, Nef, RT, and Int sequences may be altered in these ways.

The types of mutations that can be made to abrogate the in vivo function of the antigens include, but are not limited to, the following which are also described in Example 7: Mutation of Gly2 to Ala in Gag to remove a myristylation site and prevent formation of virus-like-particles (VLPs); Mutation of Gag to avoid slippage at the natural frame shift sequence to leave the conserved amino acid sequence (NFLG) intact and allow only the full-length GagPol protein product to be translated; Mutation of RT Asp185 to Ala and mutation of Asp186 to Ala to inactivate active enzyme residues. Mutation of Int Asp 64 to Ala, and mutation of Asp116 to Ala and mutation of Glu 152 to Ala to inactivate active enzyme residues.

As regards codon optimization, the nucleic acid molecules have a nucleotide sequence that encodes the antigens of the disclosure and can be designed to employ codons that are used in the genes of the subject in which the antigen is to be produced. Many viruses, including HIV and other lentiviruses, use a large number of rare codons and, by altering these codons to correspond to codons commonly used in the desired subject, enhanced expression of the antigens can be achieved. Preferably, the codons used are "humanized" codons, i.e., the codons are those that appear frequently in highly expressed human genes (Andre et al., J. Virol. 72:1497-1503, 1998) instead of those codons that are frequently used by HIV. Such codon usage provides for efficient expression of the transgenic HIV proteins in human cells. Any suitable method of codon optimization may be used. For example, codons may be optimized for human usage as illustrated in Example 8. However, any other suitable methods of codon optimization may be used. Such methods, and the selection of such methods, are well known to those of skill in the art. In addition, there are several companies that will optimize codons of sequences, such as Geneart (geneart.com). Thus, the nucleotide sequences can readily be codon optimized.

The application further encompasses nucleotide sequences encoding functionally and/or antigenically equivalent variants and derivatives of the antigens of the disclosure and functionally equivalent fragments thereof. These functionally equivalent variants, derivatives, and fragments display the ability to retain antigenic activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. Conservative amino acid substitutions are glycine/alanine; valine/isoleucine/leucine; asparagine/glutamine; aspartic acid/glutamic acid; serine/threonine/methionine; lysine/arginine; and phenylalanine/tyrosine/tryptophan. The variants have at least 50%, at least 55%, at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% homology or identity to the antigen, epitope, immunogen, peptide or polypeptide of interest.

Sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990; 87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pcarson & Lipman, Proc. Natl. Acad. Sci. USA 1988; 85: 2444-2448.

Advantageous for use is the WU-BLAST (Washington University BLAST) version 2.0 software. WU-BLAST version 2.0 executable programs for several UNIX platforms can be downloaded from ftp ://blast.wustl.edu/blast/executables. This program is based on WU-BLAST version 1.4, which in turn is based on the public domain NCBI-BLAST version 1.4 (Altschul & Gish, 1996, Local alignment statistics, Doolittle ed., Methods in Enzymology 266: 460-480; Altschul et al., Journal of Molecular Biology 1990; 215: 403-410; Gish & States, 1993;Nature Genetics 3: 266-272; Karlin & Altschul, 1993;Proc. Natl. Acad. Sci. USA 90: 5873-5877).

The various recombinant nucleotide sequences and transgenes are made using standard recombinant DNA and cloning techniques. Such techniques are well known to those of skill in the art. See for example, "Molecular Cloning: A Laboratory Manual", second edition (Sambrook et al. 1989).

The nucleotide sequences of the present invention may be inserted into "vectors." The term "vector" is widely used and understood by those of skill in the art, and as used herein the term "vector" is used consistent with its meaning to those of skill in the art. For example, the term "vector" is commonly used by those skilled in the art to refer to a vehicle that allows or facilitates the transfer of nucleic acid molecules from one environment to another or that allows or facilitates the manipulation of a nucleic acid molecule.

Any vector that allows expression of the HIV-1 Clade A transgenes may be used in accordance with the present disclosure. In certain embodiments, the HIV-1 Clade A transgenes of the present disclosure may be used *in vitro* (such as using cell-free expression systems) and/or in cultured cells grown *in vitro* in order to produce the encoded HIV-1 antigens which may then be used for various applications such as in the production of proteinaceous vaccines. For such applications, any vector that allows expression of the HIV-1 Clade A transgenes *in vitro* and/or in cultured cells may be used.

For applications where it is desired that the transgenes be expressed *in vivo,* for example when the transgenes are used in DNA or DNA-containing vaccines, any vector that allows for the expression of the HIV-1 Clade A transgenes of the present disclosure and is safe for use *in vivo* may be used. The vectors used are safe for use in humans, mammals and/or laboratory animals.

In order for the transgenes to be expressed, the protein coding sequence should be "operably linked" to regulatory or nucleic acid control sequences that direct transcription and translation of the protein. As used herein, a coding sequence and a nucleic acid control sequence or promoter are said to be "operably linked" when they are covalently linked in such a way as to place the expression or transcription and/or translation of the coding sequence under the influence or control of the nucleic acid control sequence. The "nucleic acid control sequence" can be any nucleic acid element, such as, but not limited to promoters, enhancers, IRES, introns, and other elements described herein that direct the expression of a nucleic acid sequence or coding sequence that is operably linked thereto. The term "promoter" will be used herein to refer to a group of transcriptional control modules that are clustered around the initiation site for RNA polymerase II and that when operationally linked to the protein coding sequences of the disclosure lead to the expression of the encoded protein. The expression of the transgenes can be under the control of a constitutive promoter or of an inducible promoter, which initiates transcription only when exposed to some particular external stimulus, such as, without limitation, antibiotics such as tetracycline, hormones such as ecdysone, or heavy metals. The promoter can also be specific to a particular cell-type, tissue or organ. Many suitable promoters and enhancers are known in the art, and any such suitable promoter or enhancer may be used for expression of the transgenes of the disclosure. For example, suitable promoters and/or enhancers can be selected from the Eukaryotic Promoter Database (EPDB).

The vectors should typically be chosen such that they contain a suitable gene regulatory region, such as a promoter or enhancer, such that the transgenes can be expressed.

For example, when the aim is to express the transgenes in vitro, or in cultured cells, or in any prokaryotic or eukaryotic system for the purpose of producing the protein(s) encoded by that transgene, then any suitable vector can be used depending on the application. For example, plasmids, viral vectors, bacterial vectors, protozoal vectors, insect vectors, baculovirus expression vectors, yeast vectors, mammalian cell vectors, and the like, can be used. Suitable vectors can be selected by the skilled artisan taking into consideration the characteristics of the vector and the requirements for expressing the transgenes under the identified circumstances.

When the aim is to express the transgenes in vivo in a subject, for example in order to generate an immune response against an H1V-1 antigen and/or protective immunity against HIV-1, expression vectors that are suitable for expression on that subject, and that are safe for use *in vivo,* should be chosen. For example, in some embodiments it may be desired to express the transgenes in a laboratory animal, such as for pre-clinical testing of the HIV-1 immunogenic compositions and vaccines of the invention. In other embodiments, it will be desirable to express the transgenes in human subjects, such as in clinical trials and for actual clinical use of the immunogenic compositions and vaccine of the invention. Any vectors that are suitable for such uses can be employed, and it is well within the capabilities of the skilled artisan to selelct a suitable vector. In some embodiments it may be preferred that the vectors used for these *in vivo* applications are attenuated to vector from amplifying in the subject. For example, if plasmid vectors are used, preferably they will lack an origin of replication that functions in the subject so as to enhance safety for *in vivo* use in the subject.. If viral vectors are used, preferably they are attenuated or replication-defective in the subject, again, so as to enhance safety for *in vivo* use in the subject.

In preferred embodiments viral vectors are used. Viral expression vectors are well known to those skilled in the art and include, for example, viruses such as adenoviruses, adeno-associated viruses (AAV), alphaviruses, herpesviruses, retroviruses and poxviruses, including avipox viruses, attenuated poxviruses, vaccinia viruses, and particularly, the modified vaccinia Ankara virus (MVA; ATCC Accession No. VR-1566). Such viruses, when used as expression vectors are innately non-pathogenic in the selected subjects such as humans or have been modified to render them non-pathogenic in the selected subjects. For example, replication-defective adenoviruses and alphaviruses are well known and can be used as gene delivery vectors.

In particularly preferred embodiments adenovirus vectors are used. Many adenovirus vectors are known in the art and any such suitable vector my be used. In preferred embodiments the adenovirus vector used is selected from the group consisting of the Ad5, Ad35, Ad11, C6, and C7 vectors.

The sequence of the Adenovirus 5 ("Ad5") genome has been published. (Chroboczek, J., Bieber, F., and Jacrot, B. (1992) The Sequence of the Genome of Adenovirus Type 5 and Its Comparison with the Genome of Adenovirus Type 2, Virology 186, 280-285) Ad35 vectors are described in U.S. Patent Nos. 6,974,695, 6,913,922, and 6,869,794. Ad11 vectors are described in U.S. Patent No. 6,913,922. C6 adenovirus vectors are described in U.S. Patent Nos. 6,780,407; 6,537,594; 6,309,647; 6,265,189; 6,156,567; 6,090,393; 5,942,235 and 5,833,975. C7 vectors are described in U.S. Patent No. 6,277,558.

Adenovirus vectors that arc E1-defective or deleted, E3-defective or deleted, and/or E4-defective or deleted may also be used. Certain adenoviruses having mutations in the E1 region have improved safety margin because E1-defective adenovirus mutants are replication-defective in non-permissive cells, or, at the very least, are highly attenuated. Adenoviruses having mutations in the E3 region may have enhanced the immunogenicity by disrupting the mechanism whereby adenovirus down-regulates MHC class I molecules. Adenoviruses having E4 mutations may have reduced immunogenicity of the adenovirus vector because of suppression of late gene expression. Such vectors may be particularly useful when repeated re-vaccination utilizing the same vector is desired. Adenovirus vectors that are deleted or mutated in E1, E3, E4, E1 and E3, and E1 and E4 can be used.

Furthermore, "gutless" adenovirus vectors, in which all viral genes are deleted, can also be used. Such vectors require a helper virus for their replication and require a special human 293 cell line expressing both E1a and Cre, a condition that does not exist in natural environment. Such "gutless" vectors are non-immunogenic and thus the vectors may be inoculated multiple times for re-vaccination. The "gutless" adenovirus vectors can be used for insertion of heterologous inserts/genes such as the transgenes, and can even be used for co-delivery of a large number of heterologous inserts/genes.

The application also encompasses a design that puts the Env and GRIN on separate vectors to allow assessment of whether inclusion of Env is beneficial or detrimental in terms of cell-mediated immunity (CMI) and protective efficacy. The benefits and/or detriments of Env on CMI and protective efficacy remains an open question in the HIV vaccine field.

The nucleotide sequences and vectors of the disclosure can be delivered to cells, for example if aim is to express and the HIV-1 antigens in cells in order to produce and isolate the expressed proteins, such as from cells grown in culture. For expressing the transgenes in cells any suitable transfection, transformation, or gene delivery methods can be used. Such methods are well known by those skilled in the art, and one of skill in the art would readily be able to select a suitable method depending on the nature of the nucleotide sequences, vectors, and cell types used. For example, transfection, transformation, microinjection, infection, electroporation, lipofection, or liposome-mediated delivery could be used. Expression of the antigens can be carried out in any suitable type of host cells, such as bacterial cells, yeast, insect cells, and mammalian cells. The HIV-1 Clade A antigens can also be expressed using including *in vitro* transcription/translation systems. All of such methods are well known by those skilled in the art, and one of skill in the art would readily be able to select a suitable method depending on the nature of the nucleotide sequences, vectors, and cell types used.

Following expression, the antigens can be isolated and/or purified or concentrated using any suitable technique known in the art. For example, anion or cation exchange chromatography, phosphocellulose chromatography, hydrophobic interaction chromatography, affinity chromatography, immuno-affinity chromatography, hydroxyapatite chromatography, lectin chromatography, molecular sieve chromatography, isoelectric focusing, gel electrophoresis, or any other suitable method or combination of methods can be used.

In preferred embodiments, the nucleotide sequences and/or antigens are administered *in vivo,* for example where the aim is to produce an immunogenic response in a subject. A "subject" in the context of the present invention may be any animal. For example, in some embodiments it may be desired to express the transgenes in a laboratory animal, such as for pre-clinical testing of the HIV-1 immunogenic compositions and vaccines of the invention. In other embodiments, it will be desirable to express the transgenes in human subjects, such as in clinical trials and for actual clinical use of the immunogenic compositions and vaccine of the invention. In preferred embodiments the subject is a human, for example a human that is infected with, or is at risk of infection with, HIV-1.

For such *in vivo* applications the nucleotide sequences and/or antigens are preferably administered as a component of an immunogenic composition comprising the nucleotide sequences and/or antigens in admixture with a pharmaceutically acceptable carrier. The immunogenic compositions of the invention are useful to stimulate an immune response against HIV-1 and may be used as one or more components of a prophylactic or therapeutic vaccine against HIV-1 for the prevention, amelioration or treatment of AIDS. The nucleic acids of the invention are particularly useful for providing genetic vaccines, i.e. vaccines for delivering the nucleic acids encoding the HIV-1 Clade A antigens to a subject, such as a human, such that the HIV-1 Clade A antigens are then expressed in the subject to elicit an immune response.

The compositions of the invention may be injectable suspensions, solutions, sprays, lyophilized powders, syrups, elixirs and the like. Any suitable form of composition may be used. To prepare such a composition, a nucleic acid or vector having the desired degree of purity, is mixed with one or more pharmaceutically acceptable carriers and/or excipients. The carriers and excipients must be "acceptable" in the sense of being compatible with the other ingredients of the composition. Acceptable carriers, excipients, or stabilizers are nontoxic to recipients at the dosages and concentrations employed, and include, but are not limited to, water, saline, phosphate buffered saline, dextrose, glycerol, ethanol, or combinations thereof, buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

An immunogenic or immunological composition can also be formulated in the form of an oil-in-water emulsion. The oil-in-water emulsion can be based, for example, on light liquid paraffin oil (European Pharmacopea type); isoprenoid oil such as squalane, squalene, EICOSANE™ or tetratetracontane; oil resulting from the oligomerization of alkene(s), e.g., isobutene or decene; esters of acids or of alcohols containing a linear alkyl group, such as plant oils, ethyl oleate, propylene glycol di(caprylate/caprate), glyceryl tri(caprylate/caprate) or propylene glycol dioleate; esters of branched fatty acids or alcohols, e.g., isostearic acid esters. The oil advantageously is used in combination with emulsifiers to form the emulsion. The emulsifiers can be nonionic surfactants, such as esters of sorbitan, mannide (e.g., anhydromannitol oleate), glycerol, polyglycerol, propylene glycol, and oleic, isostearic, ricinoleic, or hydroxystearic acid, which are optionally ethoxylated, and polyoxypropylene-polyoxyethylene copolymer blocks, such as the Pluronic® products, e.g., L121. The adjuvant can be a mixture of emulsifier(s), micelle-forming agent, and oil such as that which is commercially available under the name Provax® (IDEC Pharmaceuticals, San Diego, CA).

The immunogenic compositions of the invention can contain additional substances, such as wetting or emulsifying agents, buffering agents, or adjuvants to enhance the effectiveness of the vaccines (Remington's Pharmaceutical Sciences, 18th edition, Mack Publishing Company, (ed.) 1980).

Adjuvants may also be included. Adjuvants include, but are not limited to, mineral salts (e.g., AlK(SO₄)₂, AlNa(SO₄)₂, AlNH(SO₄)₂, silica, alum, Al(OH)₃, Ca₃(PO₄)₂, kaolin, or carbon), polynucleotides with or without immune stimulating complexes (ISCOMs) (e.g., CpG oligonucleotides, such as those described in Chuang, T.H. et al, (2002) J. Leuk. Biol. 71(3): 538-44; Ahmad-Nejad, P. et al (2002) Eur. J. Immunol. 32(7): 1958-68; poly IC or poly AU acids, polyarginine with or without CpG (also known in the art as IC31; see Schellack, C. et al (2003) Proceedings of the 34th Annual Meeting of the German Society of Immunology; Lingnau, K. et al (2002) Vaccine 20(29-30): 3498-508), JuvaVax™ (U.S. Patent No. 6,693,086), certain natural substances (e.g., wax D from *Mycobacterium tuberculosis*, substances found in *Cornyebacterium parvum*, *Bordetella pertussis*, or members of the genus *Brucella),* flagellin (Toll-like receptor 5 ligand; see McSorley, S.J. et al (2002) J. Immunol. 169(7): 3914-9), saponins such as QS21, QS17, and QS7 (U.S. Patent Nos. 5,057,540; 5,650,398; 6,524,584; 6,645,495), monophosphoryl lipid A, in particular, 3-de-O-acylated monophosphoryl lipid A (3D-MPL), imiquimod (also known in the art as IQM and commercially available as Aldara®; U.S. Patent Nos. 4,689,338; 5,238,944; Zuber, A.K. et al (2004) 22(13-14): 1791-8), and the CCR5 inhibitor CMPD167 (see Veazey, R.S. et al (2003) J. Exp. Med. 198: 1551-1562).

Aluminum hydroxide or phosphate (alum) are commonly used at 0.05 to 0.1% solution in phosphate buffered saline. Other adjuvants that can be used, especially with DNA vaccines, are cholera toxin, especially CTA1-DD/ISCOMs (see Mowat, A.M. et al (2001) J. Immunol. 167(6): 3398-405), polyphosphazenes (Allcock, H.R. (1998) App. Organometallic Chem. 12(10-11): 659-666; Payne, L.G. et al (1995) Pharm. Biotechnol. 6: 473-93), cytokines such as, but not limited to, IL-2, IL-4, GM-CSF, IL-12, IL-15 IGF-1, IFN-α, IFN-β, and IFN-γ (Boyer et al., (2002) J. Liposome Res. 121:137-142; WO01/095919), immunoregulatory proteins such as CD40L (ADX40; see, for example, WO03/063899), and the CD1a ligand of natural killer cells (also known as CRONY or α-galactosyl ceramide; see Green, T.D. et al, (2003) J. Virol. 77(3): 2046-2055), immunostimulatory fusion proteins such as IL-2 fused to the Fc fragment of immunoglobulins (Barouch et al., Science 290:486-492, 2000) and co-stimulatory molecules B7.1 and B7.2 (Boyer), all of which can be administered either as proteins or in the form of DNA, on the same expression vectors as those encoding the antigens of the invention or on separate expression vectors.

The immunogenic compositions can be designed to introduce the HIV-1 Clade A antigens, nucleic acids or expression vectors to a desired site of action and release it at an appropriate and controllable rate. Methods of preparing controlled-release formulations are known in the art. For example, controlled release preparations can be produced by the use of polymers to complex or absorb the immunogen and/or immunogenic composition. A controlled-release formulations can be prepared using appropriate macromolecules (for example, polyesters, polyamino acids, polyvinyl, pyrrolidone, ethylenevinylacetate, methylcellulose, carboxymethylcellulose, or protamine sulfate) known to provide the desired controlled release characteristics or release profile. Another possible method to control the duration of action by a controlled-release preparation is to incorporate the active ingredients into particles of a polymeric material such as, for example, polyesters, polyamino acids, hydrogels, polylactic acid, polyglycolic acid, copolymers of these acids, or ethylene vinylacetate copolymers. Alternatively, instead of incorporating these active ingredients into polymeric particles, it is possible to entrap these materials into microcapsules prepared, for example, by coacervation techniques or by interfacial polymerization, for example, hydroxymethylcellulose or gelatin-microcapsule and poly-(methylmethacrylate) microcapsule, respectively, in colloidal drug delivery systems (for example, liposomes, albumin microspheres, microemulsions, nano-particles and nanocapsules) or in macroemulsions. Such techniques are disclosed in New Trends and Developments in Vaccines, Voller et al. (eds.), University Park Press, Baltimore, Md., 1978 and Remington's Pharmaceutical Sciences, 16th edition.

Suitable dosages of the HIV-1 Clade A antigens, nucleic acids and expression vectors (collectively, the immunogens) in the immunogenic composition can be readily determined by those of skill in the art. For example, the dosage of the immunogens can vary depending on the route of administration and the size of the subject. Suitable doses can be determined by those of skill in the art, for example by measuring the immune response of a subject, such as a laboratry animal, using conventional immunological techniques, and adjusting the dosages as appropriate. Such techniques for measuring the immune response of the subject include but are not limited to, chromium release assays, tetramer binding assays, IFN-γ ELISPOT assays, IL-2 ELISPOT assays, intracellular cytokine assays, and other immunological detection assays, e.g., as detailed in the text "Antibodies: A Laboratory Manual" by Ed Harlow and David Lane.

When provided prophylactically, the immunogenic compositions of the invention are ideally administered to a subject in advance of HIV infection, or evidence of HIV infection, or in advance of any symptom due to AIDS, especially in high-risk subjects. The prophylactic administration of the immunogenic compositions can serve to provide protective immunity of a subject against HIV-1 infection or to prevent or attenuate the progression of AIDS in a subject already infected with HIV-1. When provided therapeutically, the immunogenic compositions can serve to ameliorate and treat AIDS symptoms and are advantageously used as soon after infection as possible, preferably before appearance of any symptoms of AIDS but may also be used at (or after) the onset of the disease symptoms.

The immunogenic compositions can be administered using any suitable delivery method including, but not limited to, intramuscular, intravenous, intradermal, mucosal, and topical delivery. Such techniques are well known to those of skill in the art. More specific examples of delivery methods are intramuscular injection, intradermal injection, and subcutaneous injection. However, delivery need not be limited to injection methods. Further, delivery of DNA to animal tissue has been achieved by cationic liposomes (Watanabe et al., (1994) Mol. Reprod. Dev. 38:268-274; and WO 96/20013), direct injection of naked DNA into animal muscle tissue (Robinson et al., (1993) Vaccine 11:957-960; Hoffman et al., (1994) Vaccine 12: 1529-1533; Xiang et al., (1994) Virology 199: 132-140; Webster et al., (1994) Vaccine 12: 1495-1498; Davis et al., (1994) Vaccine 12: 1503-1509; and Davis et al., (1993) Hum. Mol. Gen. 2: 1847-1851), or intradermal injection of DNA using "gene gun" technology (Johnston et al., (1994) Meth. Cell Biol. 43:353-365). Alternatively, delivery routes can be oral, intranasal or by any other suitable route. Delivery also be accomplished via a mucosal surface such as the anal, vaginal or oral mucosa.

Immunization schedules (or regimens) are well known for animals (including humans) and can be readily determined for the particular subject and immunogenic composition. Hence, the immunogens can be administered one or more times to the subject. Preferably, there is a set time interval between separate administrations of the immunogenic composition. While this interval varies for every subject, typically it ranges from 10 days to several weeks, and is often 2, 4, 6 or 8 weeks. For humans, the interval is typically from 2 to 6 weeks. The immunization regimes typically have from 1 to 6 administrations of the immunogenic composition, but may have as few as one or two or four. The methods of inducing an immune response can also include administration of an adjuvant with the immunogens. In some instances, annual, biannual or other long interval (5-10 years) booster immunization can supplement the initial immunization protocol.

The present methods also include a variety of prime-boost regimens, especially DNA prime-Adenovirus boost regimens. In these methods, one or more priming immunizations are followed by one or more boosting immunizations. The actual immunogenic composition can be the same or different for each immunization and the type of immunogenic composition (e.g., containing protein or expression vector), the route, and formulation of the immunogens can also be varied. For example, if an expression vector is used for the priming and boosting steps, it can either be of the same or different type (e.g., DNA or bacterial or viral expression vector). One useful prime-boost regimen provides for two priming immunizations, four weeks apart, followed by two boosting immunizations at 4 and 8 weeks after the last priming immunization. It should also be readily apparent to one of skill in the art that there are several permutations and combinations that are encompassed using the DNA, bacterial and viral expression vectors of the disclosure to provide priming and boosting regimens.

Also disclosed are methods of inducing an immune response against HIV in a subject by administering an immunogenic composition of the invention, preferably comprising an adenovirus vector containing DNA encoding one or more of the HIV-1 Clade A antigens of the invention, (preferably GRIN, GRN, or Env, or a combination thereof), one or more times to a subject wherein the HIV-1 Clade A antigen(s) are expressed at a level sufficient to induce a specific immune response in the subject. Such immunizations can be repeated multiple times at time intervals of at least 2, 4 or 6 weeks (or more) in accordance with a desired immunization regime.

The immunogenic compositions of the invention can be administered alone, or can be co-administered, or sequentially administered, with other HIV immunogens and/or HIV immunogenic compositions, e.g., with "other" immunological, antigenic or vaccine or therapeutic compositions thereby providing multivalent or "cocktail" or combination compositions and methods of employing them. Again, the ingredients and manner (sequential or co-administration) of administration, as well as dosages can be determined taking into consideration such factors as the age, sex, weight, species and condition of the particular subject, and the route of administration.

When used in combination, the other HIV immunogens can be administered at the same time or at different times as part of an overall immunization regime, e.g., as part of a prime-boost regimen or other immunization protocol. Many other HIV immunogens are known in the art, one such preferred immunogen is HIVA (described in WO 01/47955), which can be administered as a protein, on a plasmid (e.g., pTHr.HIVA) or in a viral vector (e.g., MVA.HIVA). Another such HIV immunogen is RENTA (described in PCT/US2004/037699), which can also be administered as a protein, on a plasmid (e.g., pTHr.RENTA) or in a viral vector (e.g., MVA.RENTA).

For example, one method of inducing an immune response against HIV in a human subject comprises administering at least one priming dose of an HIV immunogen and at least one boosting dose of an HIV immunogen, wherein the immunogen in each dose can be the same or different, provided that at least one of the immunogens is an HIV-1 Clade A antigen of the disclosure, a nucleic acid encoding an HIV-1 Clade A antigen of the disclosure or an expression vector, preferably an adenovirus vector, encoding an HIV-1 Clade A antigen, and wherein the immunogens are administered in an amount or expressed at a level sufficient to induce an HIV-specific immune response in the subject. The HIV-specific immune response can include an HIV-specific T-cell immune response or an HIV-specific B-cell immune response. Such immunizations can be done at intervals, preferably of at least 2-6 or more weeks.

The following non-limiting examples are given for the purpose of illustrating various embodiments of the disclosure.

### EXAMPLES

### Example 1: Consensus sequence for Gag of HIV Clade A

**Table 1**

| | Distance from consensus | Country | Year |
|---|---|---|---|
| A_consensu | 0 | | |
| A_97TZ02_1 | 0.04081 | TZ | 1997 |
| A_TZA173_1 | 0.0425 | TZ | 2001 |
| A_KNH1144_ | 0.04259 | KE | 2000 |
| A_SE7535UG | 0.04303 | UG | 1994 |
| A_KNH1211_ | 0.04463 | KE | 2000 |
| A_KSM4024_ | 0.04684 | KE | 2000 |
| A_KNH1207_ | 0.04701 | KE | 2000 |
| A_SE6594UG | 0.04709 | UG | 1993 |
| A_92UG037_ | 0.05079 | UG | 1992 |
| A_TZA195_1 | 0.05127 | TZ | 2001 |
| A_MSA4079_ | 0.05279 | KE | 2000 |
| A_TZA341_1 | 0.05523 | TZ | 2001 |
| A_MSA4072_ | 0.05583 | KE | 2000 |
| A_MSA4076_ | 0.056 | KE | 2000 |
| A_KNH1199_^{.} | 0.05687 | KE | 2000 |
| A_MSA4070_ | 0.05947 | KE | 2000 |
| A_98UG5713 | 0.06038 | UG | 1998 |
| A_KEQ23-17 | 0.06072 | KE | 1994 |
| A_KNH1209_ | 0.06101 | KE | 2000 |
| A_NKU3005_ | 0.06108 | KE | 2000 |
| A_SE7253SO | 0.06113 | SO | 1994 |
| A_98UG5713 | 0.06119 | UG | 1998 |
| A_SE8538TZ | 0.06137 | TZ | 1995 |
| A_KNH1088_ | 0.06262 | KE | 1999 |
| A_KER2008_ | 0.065 | KE | 2000 |
| A_99UGA070 | 0.06531 | UG | 1999 |
| A_KER2012- | 0.06654 | KE | 2000 |
| A_KER2009_ | 0.0674 | KE | 2000 |
| A_99UGG033 | 0.06871 | UG | 1999 |
| A_KSM4030- | 0.07026 | KE | 2000 |
| A_KSM4021- | 0.07145 | KE | 1999 |
| A_98UG5713 | 0.07189 | UG | 1998 |
| A_SE8891UG | 0.07197 | UG | 1995 |
| A_SE8131UG | 0.07462 | UG | 1995 |
| A_97TZ03_1 | 0.07653 | TZ | 1997 |
| A_KNH1135_ | 0.07687 | KE | 1999 |
| A_98UG5714 | 0.0781 | UG | 1998 |
| A_UGU455_1 | 0.08349 | UG | 1985 |
| A_MSA4069_ | 0.08867 | KE | 2000 |

The amino acid sequences of the Gag proteins of 39 non-recombinant HIV Clade A strains were analyzed. Table 1 lists the 39 strains used, and refers to each by its Genbank accession number. Table 1 also identifies the country and year of isolation of each of these 39 strains. 20 of the strains were from Kenya, 12 from Uganda, 6 from Tanzania, and 1 from Somalia. 20 of the strains were isolated between 2000 and 2002, 10 were isolated between 1997 and 1999, 6 were isolated between 1994 and 1996 and 3 were isolated before 1993.

The Gag protein sequences were aligned with spaces added to preserve alignment in regions with insertions or deletions. A 50% consensus sequence was derived. The consensus amino acid sequence is shown FIG. 1. In FIG. 1 the spaces that were added to preserve alignment in regions with insertions or deletions are represented by dashes, and the positions for which a 50% consensus was not attained are represented by an "X.

For each of the 39 sequences used to generate the consensus sequence, the "distance" of that sequence from the consensus sequence was calculated using the Dayhoff PAM250 substitution matrix, which weights substitutions according to the degree of biochemical similarity. As shown in Table 1, the distance of each strain's sequence from the consensus sequence ranged from 4 to 9%.

FIG. 2 illustrates the distance of each strain's amino acid sequence from the consensus amino acid sequence in graphical form, and identifies the four strains having sequences that are closest to the consensus sequences. These four strains are strain 97TZ02 which from a low-risk individual in the Mbeya region of southwest Tanzania in 1997 which has Genbank accession number AF361872, strain TZA173 collected from an anonymous blood donor in the Mbeya region of southwest Tanzania in 2001 which has Genbank accession number AY253305, strain KNH1144 collected from an anonymous blood donor in southern Kenya in 2000 which has Genbank accession number AF4587006, and strain SE7535 collected in 1994 in Sweden from an individual thought to have been infected in Uganda which has Genbank accession number AF069671.

### Example 2: Consensus sequence for Pol of HIV Clade A

The amino acid sequences of the Pol proteins of 36 non-recombinant HIV Clade A strains were analyzed. Table 2 lists the 36 strains used, and refers to each by its Genbank accession number. Table 2 also identifies the country and year of isolation of each of these 36 strains. 20 of the strains were from Kenya, 9 from Uganda, 6 from Tanzania, and 1 from Somalia. 19 of the strains were isolated between 2000 and 2002, 10 were isolated between 1997 and 1999, 4 were isolated between 1994 and 1996 and 3 were isolated before 1993.

The Pol protein sequences were aligned. There were no insertions or deletions. A 50% consensus sequence was derived. The consensus amino acid sequence is shown FIG. 3. In FIG. 3 the positions for which a 50% consensus was not attained are represented by an "X. There were 4 such positions out of 947 amino acid residues. For each of the 36 sequences used to generate the consensus sequence, the "distance" of that sequence from the consensus sequence was calculated using the Dayhoff PAM250 substitution matrix, which weights substitutions according to the degree of biochemical similarity. As shown in Table 2, the distance of each strain's sequence from the consensus sequence ranged from 1.5 to 4.8%.

**Table 2**

| | Distance from consensus | Country | Year |
|---|---|---|---|
| A_pol.cons | 0 | | |
| A_MSA4070_ | 0.01479 | KE | 2000 |
| A_SE7253SO | 0.01582 | SO | 1994 |
| A_SE8538TZ | 0.01898 | TZ | 1995 |
| A_KER2012- | 0.02329 | KE | 2000 |
| A_97TZ02_3 | 0.0235 | TZ | 1997 |
| A_KEQ23-17 | 0.02445 | KE | 1994 |
| A_KNH1211_ | 0.02449 | KE | 2000 |
| A_TZA341_3 | 0.0246 | TZ | 2001 |
| A_KSM4024_ | 0.02528 | KE | 2000 |
| A_97TZ03_3 | 0.02544 | TZ | 1997 |
| A_KNH1088_ | 0.02544 | KE | 1999 |
| A_MSA4076_ | 0.02564 | KE | 2000 |
| A_KNH1207_ | 0.0265 | KE | 2000 |
| A_NKU3005_ | 0.02661 | KE | 2000 |
| A_TZA173_3 | 0.02756 | TZ | 2001 |
| A_MSA4079_ | 0.02762 | KE | 2000 |
| A_KER2009_ | 0.02765 | KE | 2000 |
| A_TZA195_3 | 0.02881 | TZ | 2001 |
| A_KSM4021- | 0.02881 | KE | 1999 |
| A_SE7535UG | 0.02883 | UG | 1994 |
| A_MSA4069_ | 0.02886 | KE | 2000 |
| A_SE6594UG | 0.02889 | UG | 1993 |
| A_98UG5713 | 0.02975 | UG | 1998 |
| A_KNH1135_ | 0.0299 | KE | 1999 |
| A_92UG037_ | 0.02993 | UG | 1992 |
| A_KNH1209_ | 0.03202 | KE | 2000 |
| A_99UGG033 | 0.03291 | UG | 1999 |
| A_KER2008_ | 0.03294 | KE | 2000 |
| A_KSM4030- | 0.0343 | KE | 2000 |
| A_KNH1199_ | 0.03439 | KE | 2000 |
| A_99UGA070 | 0.03537 | UG | 1999 |
| A_MSA4072_ | 0.03625 | KE | 2000 |
| A_KNH1144_ | 0.03863 | KE | 2000 |
| A_98UG5713. | 0.04178 | UG | 1998 |
| A_UGU455_3 | 0.04294 | UG | 1985 |
| A_98UG5713 | 0.04808 | UG | 1998 |

FIG. 4 illustrates the distance of each strain's amino acid sequence from the consensus amino acid sequence in graphical form, and identifies the three strains having sequences that are closest to the consensus sequences. These three strains are strain MSA4070 from an anonymous blood donor in Southern Kenya in 2000, strain SE7235SO which was collected in 1994 from an individual in Sweden thought to have been infected in Somalia, and strain SE8538 which was collected in 1995 from an individual in Sweden thought to have been infected in Tanzania.

### Example 3: Consensus sequence for Env of HIV Clade A

**Table 3**

| | Dist from A.cons | country | year |
|---|---|---|---|
| A.cons | 0 | | |
| A_KEQ23-17 | 0.06307 | KE | 1994 |
| A_TZA341_1 | 0.06413 | TZ | 2001 |
| A_KNH1088_ | 0.06524 | KE | 1999 |
| A_KNH1209_ | 0.0699 | KE | 2000 |
| A_KNH1144_ | 0.07088 | KE | 2000 |
| A_99UGA070 | 0.07365 | UG | 1999 |
| A_MSA4072_ | 0.07516 | KE | 2000 |
| A_KSM4021_ | 0.0778 | KE | 1999 |
| A_97TZ02_1 | 0.07825 | TZ | 1997 |
| A_KNH1199_ | 0.07883 | KE | 2000 |
| A_MSA4079_ | 0.07944 | KE | 2000 |
| A_SE7535UG | 0.08375 | UG | 1994 |
| A_SE8538TZ | 0.08432 | TZ | 1995 |
| A_98UG5713 | 0.08462 | UG | 1998 |
| A_97TZ03_1 | 0.08541 | TZ | 1997 |
| A_MSA4070_ | 0.0874 | KE | 2000 |
| A_NKU3005_ | 0.0884 | KE | 2000 |
| A_TZA173_1 | 0.09046 | TZ | 2001 |
| A_KNH1207_ | 0.09106 | KE | 2000 |
| A_TZA195_1 | 0.09389 | TZ | 2001 |
| A_MSA4076_ | 0.09517 | KE | 2000 |
| A_92UG037_ | 0.098 | UG | 1992 |
| A_98UG5714 | 0.09816 | UG | 1998 |
| A_SE7253SO | 0.09886 | SO | 1994 |
| A_KER2012- | 0.09984 | KE | 2000 |
| A_98UG5713 | 0.10139 | UG | 1998 |
| A_SE6594UG | 0.10195 | UG | 1993 |
| A_SE8891UG | 0.10225 | UG | 1995 |
| A_UGU455_1 | 0.10314 | UG | 1985 |
| A_KER2009_ | 0.10338 | KE | 2000 |
| A_KNH1211_ | 0.11319 | KE | 2000 |
| A_SE8131UG | 0.11321 | UG | 1995 |
| A_MSA4069_ | 0.11507 | KE | 2000 |
| A_99UGG033 | 0.11653; | UG | 1999 |
| A_KNH1135 | 0.117131 | KE | 1999 |
| A_KER2008 | 0.12689 | KE | 2000 |

The amino acid sequences of the Env proteins of 36 non-recombinant HIV Clade A strains were analyzed. Table 3 lists the 36 strains used, and refers to each by its Genbank accession number. Table 3 also identifies the country and year of isolation of each of these 36 strains. 18 of the strains were from Kenya, 11 from Uganda, 6 from Tanzania, and 1 from Somalia. 17 of the strains were isolated between 2000 and 2002, 10 were isolated between 1997 and 1999, 6 were isolated between 1994 and 1996 and 3 were isolated before 1993.

The Env protein sequences were aligned with spaces added to preserve alignment in regions with insertions or deletions. There were many regions with extensive heterogeneity in the length of insertions/deletions. A 50% consensus sequence was derived. The consensus amino acid sequence is shown FIG. 5. In FIG. 5 the spaces that were added to preserve alignment in regions with insertions or deletions are represented by dashes, and the positions for which a 50% consensus was not attained are represented by an "X". There were many amino acid positions for which a 50% consensus was not attained.

For each of the 36 sequences used to generate the consensus sequence, the "distance" of that sequence from the consensus sequence was calculated using the Dayhoff PAM250 substitution matrix, which weights substitutions according to the degree of biochemical similarity. As shown in Table 3, the distance of each strain's sequence from the consensus sequence ranged from 6.3 to 12.7%.

FIG. 6 illustrates the distance of each strain's amino acid sequence from the consensus amino acid sequence in graphical form, and identifies the three strains having sequences that are closest to the consensus sequences. These three strains were KEQ23 from a CSW in Kenya in 1994 (what is a CSW), TZA341 which was from an anonymous blood donor in Tanzania in 2002, and KNH1088 which was from an anonymous blood donor in Kenya in 1999.

### Example 4: Consensus sequence for Nef of HIV Clade A

The amino acid sequences of the Nef proteins of 38 non-recombinant HIV Clade A strains were analyzed. Table 4 lists the 38 strains used, and refers to each by its Genbank accession number. The country and year of isolation of each of these 38 strains are described in Tables 1-3 in the previous Examples. More than half of the strains were from Kenya, with a substantial portion coming from Uganda, and a few strains coming from Tanzania. About half of the strains were isolated between 2000 and 2002.

**Table 4**

| | A.cons |
|---|---|
| A_MSA4070 | 0.0318 |
| A_KNH1211 | 0.04807 |
| A_97TZ03_1 | 0.0535 |
| A_99UGA070 | 0.05354 |
| A_SE8891UG | 0.05383 |
| A_KEQ23-17 | 0.06476 |
| A_98UG5713 | 0.07043 |
| A_NKU3005_ | 0.0709 |
| A_SE7535UG | 0.07117 |
| A_98UG5714 | 0.07613 |
| A_SE6594UG | 0.07634 |
| A_TZA341_1 | 0.0805 |
| A_MSA4069 | 0.08097 |
| A_KNH1199 | 0.08213 |
| A_97TZ02_1 | 0.08276 |
| A_KSM4030- | 0.08704 |
| A_KSM4021- | 0.08795 |
| A_MSA4076 | 0.08873 |
| A_KNH1209 | 0.0899 |
| A_KER2012- | 0.09224 |
| A_KNH1144 | 0.09577 |
| A_KER2008 | 0.09703 |
| A_MSA4072 | 0.09892 |
| A_98UG5713 | 0.09892 |
| A_99UGG033 | 0.09967 |
| A_KNH1088 | 0.10303 |
| A_92UG037 | 0.10654 |
| A_SE8538TZ | 0.10996 |
| A_KER2009 | 0.1102 |
| A_MSA4079 | 0.11083 |
| A_KSM4024 | 0.11126 |
| A_SE8131UG | 0.11326 |
| A_SE7253SO | 0.11453 |
| A_KNH1207 | 0.11549 |
| A_TZA173_1 | 0.13766 |
| A_98UG5713 | 0.1399 |
| A_UGU455_1 | 0.15688 |
| A_KNH1135 | 0.16076 |
| A.cons | 0 |

The Nef protein sequences were aligned with spaces added to preserve alignment in regions with insertions or deletions. A 50% consensus sequence was derived. The consensus amino acid sequence is shown FIG. 7. In FIG. 7 the spaces that were added to preserve alignment in regions with insertions or deletions are represented by dashes, and the positions for which a 50% consensus was not attained are represented by an "X". There were six amino acid positions for which a 50% consensus was not attained.

For each of the 38 sequences used to generate the consensus sequence, the "distance" of that sequence from the consensus sequence was calculated using the Dayhoff PAM250 substitution matrix, which weights substitutions according to the degree of biochemical similarity. As shown in Table 4, the distance of each strain's sequence from the consensus sequence ranged from 3.2 to 16.1% with a mean distance of 9.3%.

FIG. 8 illustrates the distance of each strain's amino acid sequence from the consensus amino acid sequence in graphical form, and identifies the five strains having sequences that are closest to the consensus sequences. These five strains were MSA4070 and KNH1211, both of which were from anonymous donors in southern Kenya and were collected in 2000, 97TZ03 from a low-risk individual in the Mbeya region of southwest Tanzania which was collected in 1997, and UGA070 and SE8891 both of which were from individuals in Uganda and were collected in 1999 and 1995, respectively.

### Example 5. Strains of HIV Clade A strains that are closest to the HIV clade A consensus sequences.

As described in Examples 1 to 4 above, and as summarized in Table 5, the strains of HIV Clade A having Gag, Pol, Env and Nef sequences that were most similar to the consensus sequences of each of these proteins were identified. In addition, the strains that were overall closest to the consensus sequence were identified by ranking each of the strains according to its closeness to the consensus sequence of a particular protein wherein the strain ranked number 1 was that whose sequence for that protein was closest to that of the consensus sequence, and then summing the rankings for each strain across all four of the proteins (i.e. Gag, Pol, Env, and Nef). The six strains that were overall closest to the consensus sequence across all four of the proteins studied are listed below in Table 6. It can be seen that strain 97TZ02 has a sequence which is overall closest to the consensus sequences of each of the Gag, Pol, Env and Nef genes.

**Table 5**

| Gag | Pol | Env | Nef |
|---|---|---|---|
| 97TZ02 | MSA4070 | KEQ23 | MSA4070 |
| TZA173 | SE7245SO | TZA341 | KNH1211 |
| KNH1144 | SE8538 | KNH1088 | 97TZ03 |
| SE7535UG | | | 99UGA070 |
| | | | SE8891UG |

**Table 6**

| | gag | pol | env | nef | sum |
|---|---|---|---|---|---|
| A_97TZ02_1 | 1 | 5 | 9 | 15 | 30 |
| A_KEQ23-17 | 18 | 6 | 1 | 6 | 31 |
| A_MSA4070_ | 16 | 1 | 16 | 1 | 34 |
| A_TZA341_1 | 12 | 8 | 2 | 12 | 34 |
| A_SE7535UG | 4 | 20 | 12 | 9 | 45 |
| A_KNH1211_ | 5 | 7 | 31 | 2 | 45 |

### Example 6. Construction of GRIN, GRN, and Env Transgenes.

Transgene constructs were made using HIV Clade A protein sequences derived from the most recently identified circulating HIV-1 field isolates that were the closest match to the HIV Clade A consensus sequence for each such protein. This strategy was developed in order to maximize the biological relevance of the HIV clade A sequences used. It should be understood that other sequences, i.e. sequences other than the specific sequences described in this example, can also be used in accordance with the disclosure. If other sequences are used it is preferred that the sequences are selected such that they are derived from recent field isolates and have sequences that are close to the HIV Clade A consensus sequences described herein, or to HIV Clade A consensus sequences that may be generated in the future.

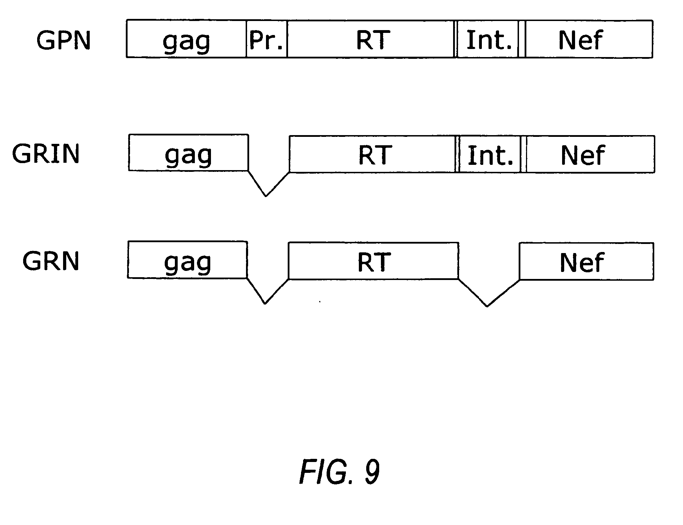

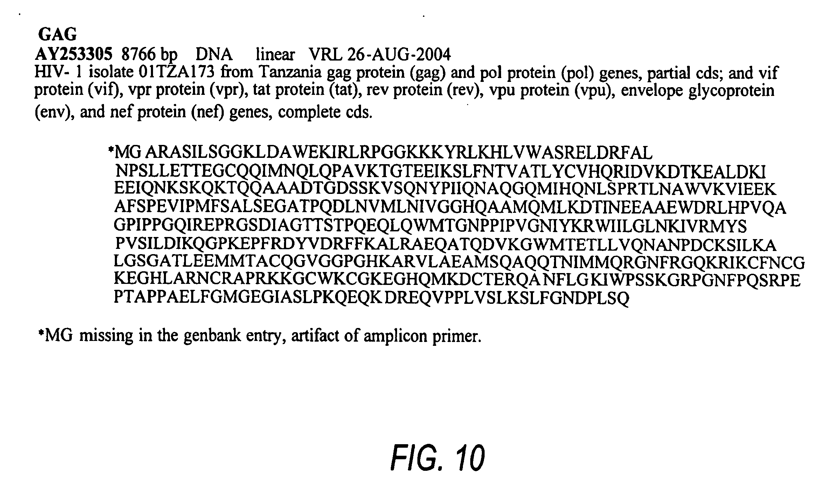

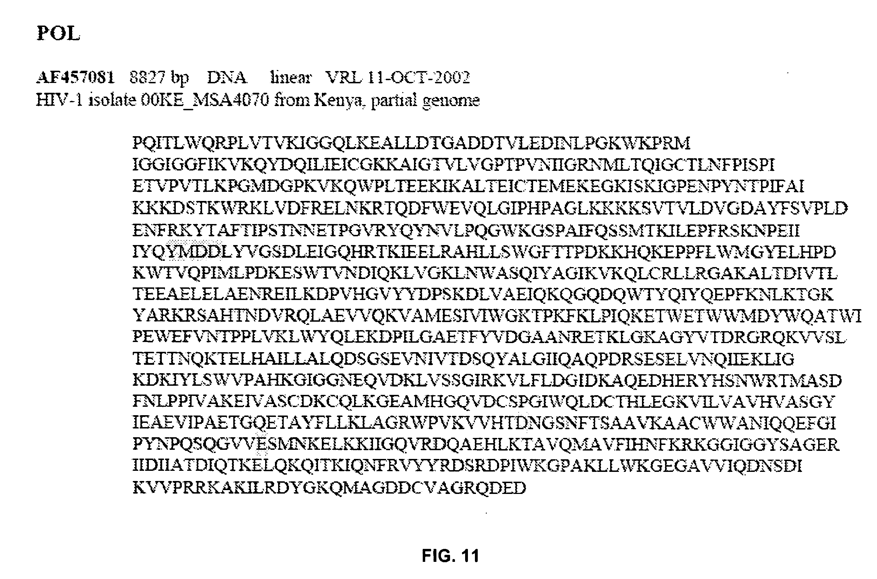

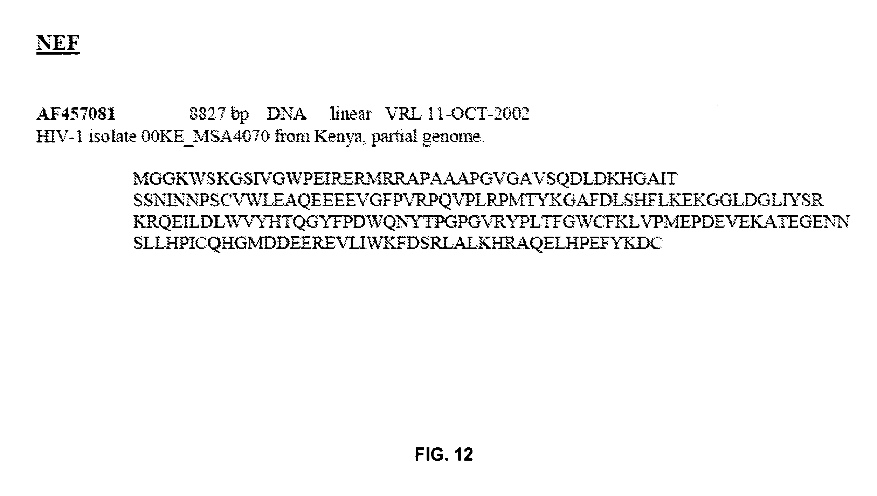

Constructs referred to as GRIN and GRN were made. The GRIN construct contained HIV Clade A sequences encoding the Gag, Pol (RT and Integrase) and Nef proteins. The GRN transgene contained sequences encoding the Gag, RT and Nef proteins. The GRIN and GRN constructs are represented schematically in FIG. 9. The GRIN and GRN transgenes were made using the Gag protein sequence from strain TZA173 having Genbank accession number AY253305, the Pol (comprising both RT and Int sequences) sequence from strain MSA4070 having Genbank accession number AF457081, and the Nef sequence from strain MSA4070 having Genbank accession number AF457081. These sequences were selected because they were from the most recently identified circulating HIV-1 field isolates that had the closest match to the consensus sequence for each of Gag, Pol, and Nef, respectively. These sequences are illustrated in FIGS. 10, 11, and 12, respectively.

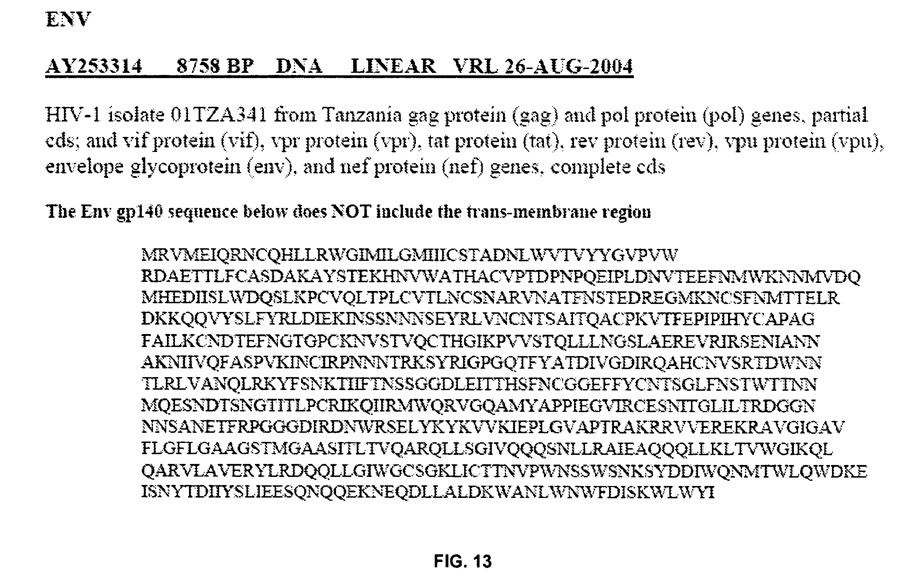

An Env construct was also made containing the Env coding sequence from the most recently identified circulating HIV-1 field isolate that had the closest match to the consensus Env sequence, i.e. the Env protein sequence from strain TZA341 having Genbank accession number AY253314. This sequence is illustrated in FIG. 13.

All sequences were then codon optimized for human expression by GeneArt (Germany). The optimized gene sequences allow high level and stable protein expression in humans or other mammalian cells. Further details of the codon optimization process are provided in Example 8. The transgenes were also engineered to incorporate specific mutations or arranged in a specific order to abrogate the normal function of the gene products *in vivo.* The details of these mutations, and the biological effects of each, are described in Example 7 below.

For the GRIN and GRN transgenes, the coding sequences for each of the Gag, Pol (RT & Int) or RT, and Nef proteins were joined in-frame such that each of the transgene constructs (i.e. either GRIN or GRN) encoded a single fusion protein. Blast searches were performed to ensure that no neoepitopes were formed at the junctions. Although not used in the present example, it should be noted that it is also possible to insert spacer sequences between the sequences coding for the individual components of the final fusion protein to allow optimal protein domain folding, for example a spacer region may be added between Gag and Pol to allow the protein domains to fold in a more native conformation. Also, unique restriction sites were added at the 5" and 3' ends of each sequence in order to facilitate the joining together of each sequence (for example, the joining of the 5" end ofNef to the 3' end of Pol, etc.).

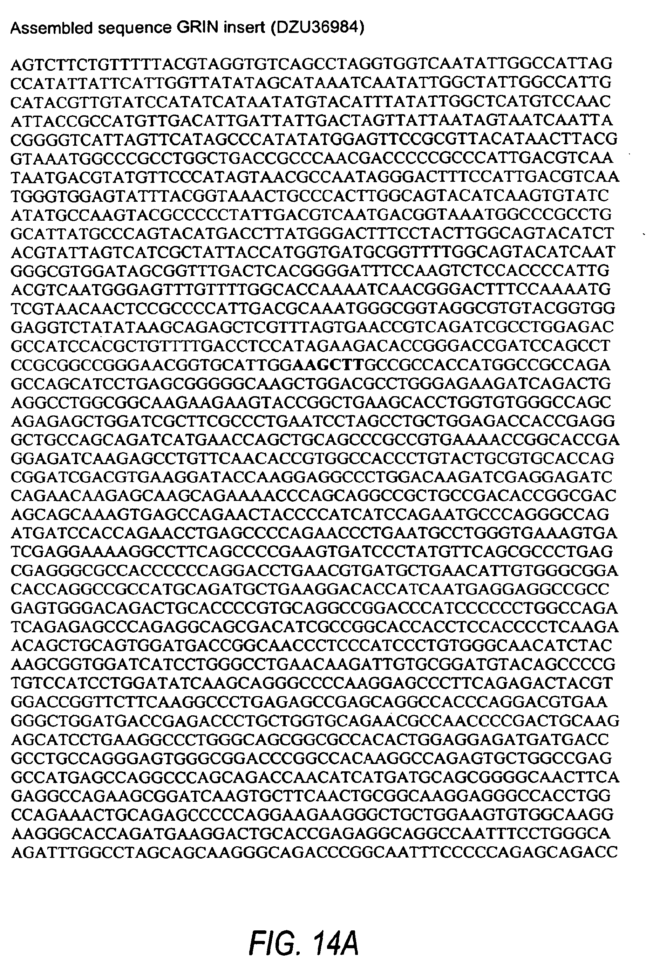

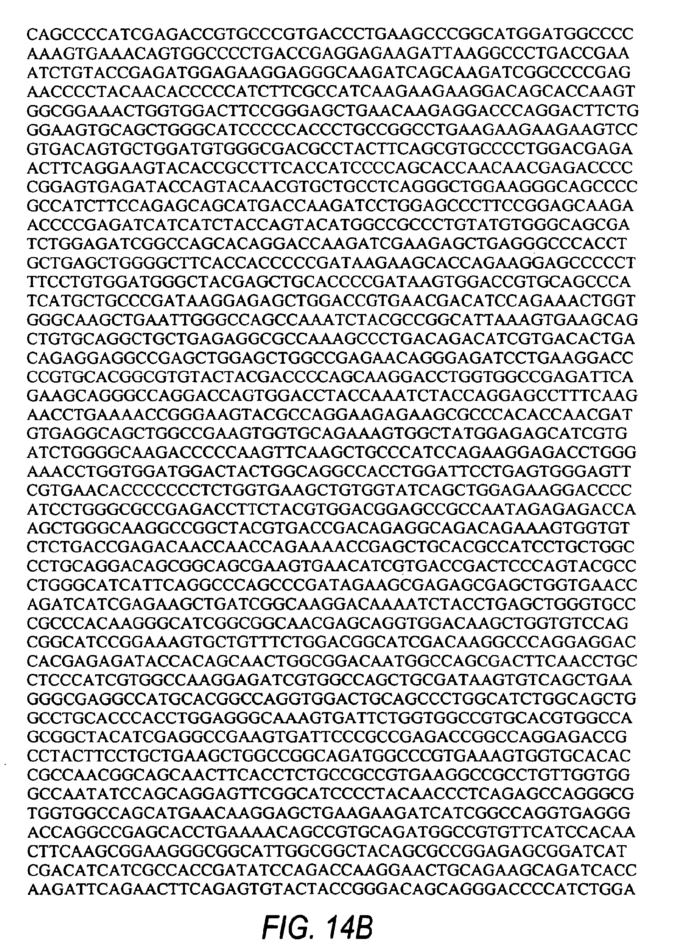

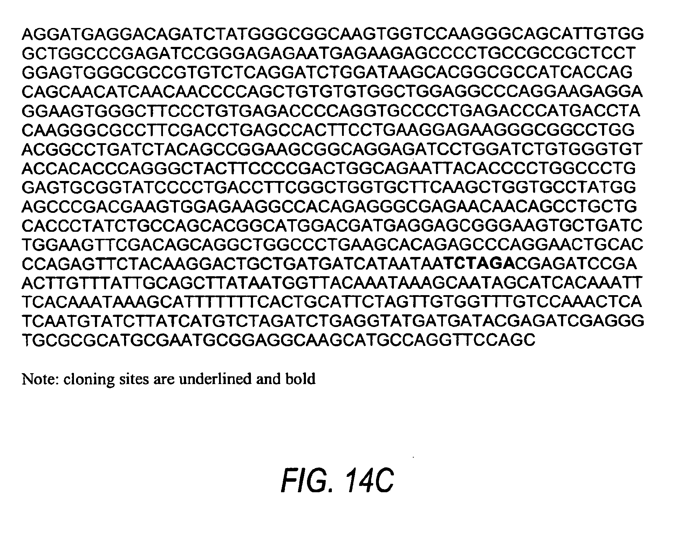

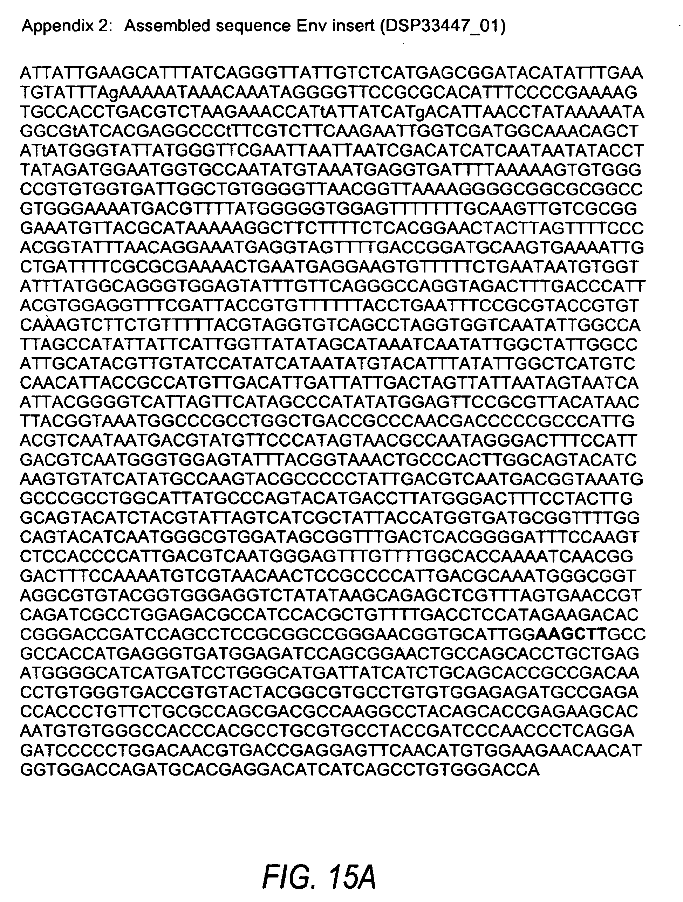

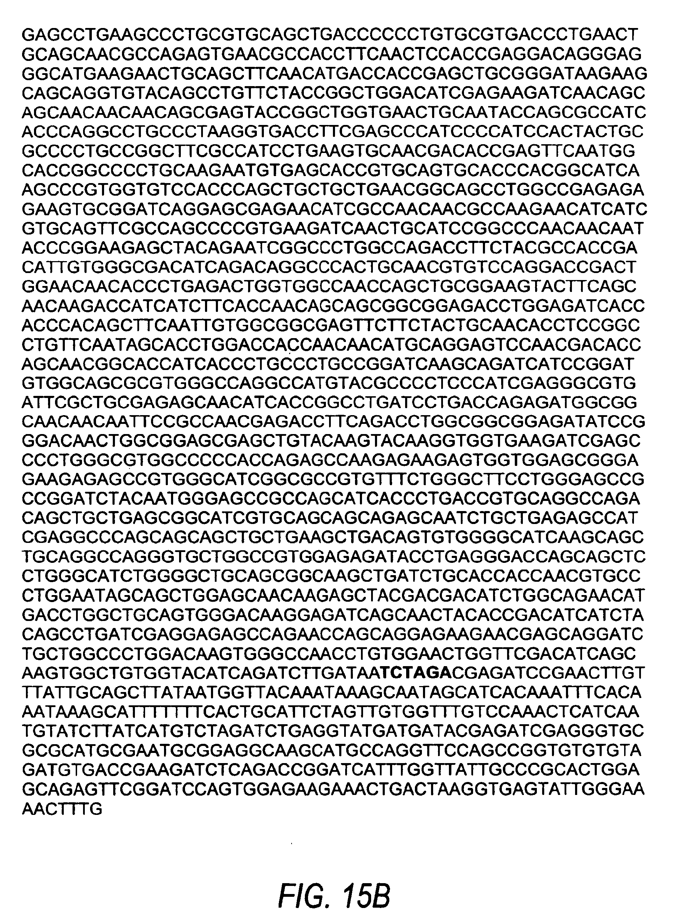

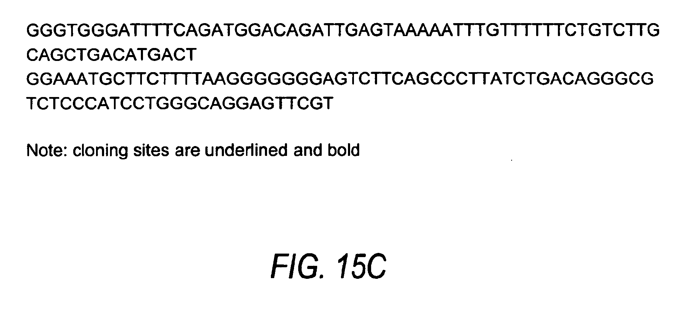

For use *in vivo* the GRIN, GRN and Env transgenes were inserted into either the Ad5, Ad35, Ad11, C6, or C7 adenovirus vectors. In order to facilitate cloning into theses vectors unique restriction sites were added at the 5" and 3' ends of the GRIN, GRN, or Env constructs. FIGS. 14A-14C provides the sequence of GRIN as inserted into the Ad35 vector, and shows the restriction sites used to clone the GRIN sequence into the Ad35 vector (underlined and in bold typeface). The sequence shown also includes the CMV promoter sequence upstream of the GRIN sequence. FIGS. 15A-15B provides the sequence of Env as inserted into the Ad35 vector, and shows the restriction sites used to clone the Env sequence into the Ad35 vector (underlined and in bold typeface). The sequence shown also includes the CMV promoter sequence upstream of the Env sequence.

Standard recombinant DNA and cloning techniques were used to generate all of the above constructs. Such techniques are well known to those of skill in the art. See for example, "Molecular Cloning: A Laboratory Manual", second edition (Sambrook et al. 1989).

### Example 7: Mutations to abrogate normal in vivo function of HIV Clade A proteins

Table 7 summarizes the mutations engineered into the GRIN and GRN sequences to abrogate the *in vivo* function of their gene products. These mutations were made using standard recombinant DNA techniques. Such techniques are well known to those of skill in the art.

**Table 7**

| **Design** | **Gene** | **Mutation/Rationale** |
|---|---|---|
| Mutation | gag | Gly2 → Ala : Removes myristylation site preventing VLP formation. |
| *Mutation* | *gag* | *To avoid slippage at the natural frame shift sequence, the DNA sequence was mutated in a manner that leaves the conserved amino acid sequence (NFLG) intact and allows only, the full-length GagPol protein product to be translated.* |
| Mutation | RT | Asp185 → Ala & Asp186 → Ala: Inactivates active enzyme residues. |
| Mutations | Integrase (IN) | Asp 64 → Ala, Asp116 → Ala & Glu 152 → Ala: Inactivates active enzyme residues. |
| No change | Nef | Fusion of nef N-terminus to IN C-terminus prevents myristylation and membrane targeting abrogating nef function. |

Gag protein is expressed as a 55-kDa polyprotein precursor (Pr55*^{gag}*), and is cleaved by the HIV-1 viral protease. Four major viral proteins result from the cleavage; Matrix (MA), Capsid (CA), Nucleocapsid (NC), and p6; as well as two spacer polypeptides p2 and p1, which represent sequences between CA and NC and between NC and p6, respectively.

MA plays a key role in several steps in virus replication, including the critical mediation of viral particle assembly and budding from the cell plasma membrane through the formation of virus-like particles (VLPs) (See Gheysen, D., E. Jacobs, F. de Foresta, D. Thiriart, M. Francotte, D. Thines, and M. De Wilde. (1989). Assembly and release of HIV-1 precursor pr55gag virus-like particles from recombinant baculovirus-infected cells. Cell 59:103-112).

Both Pr55^{gag} and the MA (p17) are myristylated, i.e. amide bond formation to myristic acid. See Veronese di Marzo, F., Copeland, T. D., Oroszlan, S., Gallo, R. C. & Sarngadharan, M. G. (1988). J. Virol. 62, 795-801. See also section on Nef within Example 7 for a full description of myristylation process. Different HIV-1 isolates demonstrate that the myristyl-acceptor is the N-terminal glycine residue (Gly2). See Bryant & Ratner. (1990). Myristoylation-dependent replication and assembly of human immunodeficiency virus 1. Proc. Nadl. Acad. Sci. USA; 87: 523-527.

Bryant and Ratner (1990) demonstrated that substitution of Gly2 with Ala eliminated virus replication of an HIV-1 clone. The Pr55^{gag}, deficient of the myristyl-acceptor glycine, accumulated in infected Hela cells and was not processed into mature virion capsid. It was concluded that myristylation of the Gly2 is required for stable plasma membrane association and subsequent assembly of virions. Other groups have similarly demonstrated the importance of the mystriylation of Gly2 in the MA. See Göttlinger H G, Sodroski J G, Haseltine W A. (1989). Role of capsid precursor processing and myristoylation in morphogenesis and infectivity of human immunodeficiency virus type 1. Proc Natl Acad Sci USA; 86:5781-5785, and Paul Spearman, Jaang-Jiun Wang, Nancy Vander Heyden and Lee Ratner. (1994). Identification of Human Immunodeficiency Virus Type 1 Gag Protein Domains Essential to Membrane Binding and Particle Assembly. J. Virol; 68 (5): 3232-3242.

If the myristyl-acceptor N-terminal glycine (Gly2) in MA is mutated, membrane binding is abrogated and particle assembly is prevented. Thus, clade A Gag is engineered to change Gly2 → Ala. This results in the loss of the Gag biological function.

Reverse transcriptase (RT) is a viral enzyme essential for replication. RT converts incoming viral RNA⁺ into dsDNA, catalyzed by the RNA- and DNA-dependent polymerase and RNase H activities of the enzyme. RT is a heterodimer composed of p66 and p51 subunit proteins. See Alfredo Jacobo-Molina et al. (1993). Crystal structure of human immunodeficiency virus type 1 reverse transcriptase complexed with double-stranded DNA at 3.0 A resolution shows bent DNA. Proc. Natl. Acad. Sci. USA; 90: 6320-6324. p66 has two domains, the polymerase and RNase H. p51 has the same polymerase domain.

The catalytically essential Asp-110, Asp-185, and Asp-186 residues are located in the highly conserved DNA polymerase active site. These three residues, termed the "the catalytic triad" are thought to bind the divalent cations necessary for catalysis function. See Alfredo Jacobo-Molina et al. (1993). Crystal structure of human immunodeficiency virus type 1 reverse transcriptase complexed with double-stranded DNA at 3.0 A resolution shows bent DNA. Proc. Natl. Acad. Sci. USA; 90: 6320-6324.

The mutation of the aspartic acids at residues 185 and 186 into either asparagine or glutamate have been demonstrated to result in mutant proteins which were catalytically inactive. See Lowe DM, Parmar V, Kemp SD, Larder BA. (1991). Mutational analysis of two conserved sequence motifs in HIV-1 reverse transcriptase. FEBS Lett.; 6;282(2):231-4.

Mutation of Asp 185 → Ala & Asp 186 → Ala in the Clade A RT will inactivate the RT polymerase enzyme by disrupting the "catalytic triad". This will eliminate the biological function of the Clade A RT.

Proviral cDNA generated by RT is integrated into the host cell genome through the action of the viral Integrase (Int) enzyme. Int contains a DNA recombinase domain that catalyzes two distinct endonucleolytic reactions. The first reaction, 3' processing, removes dinucleotides from each end of the cDNA producing two-nucleotide 5' extensions at both ends. In the second reaction, Int non-specifically cleaves the host cell DNA and joins the free 3' groups of the cDNA termini to the 5' groups of the cleaved host cell DNA. Cellular enzymes repair gaps resulting in a fully integrated viral genome into the host cell DNA. See Coffin JM. Retroviridae and their Replication. Chapter 27. p645-708 & Wong-Staal F. Human Immunodeficiency Viruses and Their Replication. Chapter 28. p709-723. In Fields, BN. & Knipe DM. 2nd Edition Fundamental Virology. Raven Press. See also Engelman A, Mizuuchi K, Craigie R. (1991). HIV-1 DNA integration: mechanism of viral DNA cleavage and DNA strand transfer. Cell; 67(6):1211-1221. The catalytic domain, residues 50 to 212, contain a triad of residues Asp-64, Asp-116, and Glu-152 (termed the D,D-35-E motif) that compromises the enzyme active site. See Esposito, D., and R. Craigie. (1999). HIV integrase structure and function. Adv. Virus Res. 52:319-333. See also Khan, E., J. P. G. Mack, R. A. Katz, J. Kulkosky, and A. M. Skalka. (1991). Retroviral integrase domains: DNA binding and the recognition of LTR sequences. Nucleic Acids Res. 19:851-860.

Through a variety of techniques, groups have demonstrated the abrogation of endonuclease and/or integration function of IN through site directed mutation of Asp-64, Asp-116, and Glu-152 residues in the D,D-35-E motif. See Drelich M, Wilhelm R, Mous J. (1992). Identification of amino acid residues critical for endonuclease and integration activities of HIV-1 IN protein in vitro. Virology;188(2):459-468. See also LaFemina RL, Schneider CL, Robbins HL, Callahan PL, LeGrow K, Roth E, Schleif WA, Emini EA. (1992). Requirement of active human immunodeficiency virus type 1 integrase enzyme for productive infection of human T-lymphoid cells. J Virol; 66(12):7414-7419. See also Leavitt AD, Shiue L, Varmus HE. (1993). Site-directed mutagenesis of HIV-1 integrase demonstrates differential effects on integrase functions in vitro. J Biol Chem; 268(3):2113-2119.

Mutation of Asp-64→ Ala, Asp-116→ Ala, and Glu-152→ Ala in the Clade A Int will inactivate the Int active enzyme by disrupting the critical D,D-35-E motif. This will eliminate the biological function of Clade A Int.

The Negative factor (Nef) protein (27-kDa) is the earliest viral protein to accumulate in the newly infected cell. See Haseltine, W. (1991). Molecular biology of the human immunodeficiency virus type 1. FASEB. Vol 5. 2349-2360. Through myristylation, Nef is able to localize on the cytosol side of the cell membrane. See Yu G, Felsted RL. (1992). Effect of myristoylation on p27 nef subcellular distribution and suppression of HIV-LTR transcription. Virology. 187(1):46-55. See also Kaminchik, J., N. Bashan, A. Itach, N. Sarver, M. Gorecki, and A. Panet. (1991). Genetic characterization of human immunodeficiency virus type 1 nef gene products translated in vitro and expressed in mammalian cells. J. Virol. 65:583-588.Myristylation of proteins is a co-translational event and involves the transfer of myristate from myristyl-Coenzyme A to the amino-terminal motif MG*XXX* of proteins by the enzyme N-myristyl transferase (NMT). See Towler, D. A., S. P. Adams, S. R. Eubanks, D. S. Towery, E. Jackson-Machelski, L. Glaser & J. I. Gordon (1987). Purification and characterization of yeast myristoyl CoA:protein N- myristoyltransferase. Proc Natl Acad Sci USA 84:2708-2712. The lead methionine of the polypeptide is cleaved by the methionine amino peptidase during translation and NMT recognizes the newly generated terminal amino group of glycine of the emerging peptide after approximately twenty residues are free of the ribosome. NMT transfers myristate to the glycine residue (the myristyl-acceptor) and myristylation is completed. Replacement of the penultimate glycine myristyl-acceptor with any other amino acid residue inhibits myristylation. See Towler, D. A., S. R. Eubanks, D. S. Towery, S. P. Adams & L. Glaser (1987). Amino-terminal processing of proteins by N-myristoylation. Substrate specificity of N-myristoyl transferase. J Biol Chem 262:1030-1036.

Nef is a multifunctional protein able to modulate a number of surface molecules of the infected cell, such as CD4 (see Garcia, J. V., and A. D. Miller. (1991). Serine phosphorylation-independent downregulation of cell-surface CD4 by nef. Nature 350:508-511; and Mariani R and Skowronski J. (1993). CD4 down-regulation by nef alleles isolated from human immunodeficiency virus type 1-infected individuals Proc. Natl. Acad. Sci. USA. Vol. 90, pp. 5549-5553; and Aiken C, Konner J, Landau NR, Lenburg ME, Trono D (1994). Nef induces CD4 endocytosis: requirement for a critical dileucine motif in the membrane-proximal CD4 cytoplasmic domain. Cell. 11;76(5):853-64), CD28 (see Swigut, T., N. Shohdy, and J. Skowronski. (2001). Mechanism for down-regulation of CD28 by Nef. EMBO J. 20:1593-1604), MHC-I (see Schwartz, O., V. Marechal, S. Le Gall, F. Lemonnier, and J. M. Heard. (1996). Endocytosis of major histocompatibility complex class I molecules is induced by the HIV-1 Nef protein. Nat. Med. 2:338-342), the macrophage-expressed MHC 1b protein HFE (see Drakesmith H, Chen N, Ledermann H, Screaton G, Townsend A, Xu XN. (2005). HIV-1 Nef down-regulates the hemochromatosis protein HFE, manipulating cellular iron homeostasis. Proc Natl Acad Sci USA. 102(31):11017-22), MHC-II (see Stumptner-Cuvelette, P., S. Morchoisne, M. Dugast, S. Le Gall, G. Raposo, O. Schwartz, and P. Benaroch. (2001). HIV-1 Nef impairs MHC class II antigen presentation and surface expression. Proc. Natl. Acad. Sci. USA 98:12144-12149), as well as disrupt signal transduction pathways (see Tolstrup, M., L. Ostergaard, A. L. Laursen, S. F. Pedersen, and M. Duch. (2004). HIV/SIV escape from immune surveillance: focus on Nef. Curr. HIV Res. 2:141-151) via association with multiple kinases and other cell surface proteins at the cell membrane. The mechanisms of these actions and the nef motifs involved remain to be fully elucidated.

Specifically, a Nef mutant with deletion of the 19 N-terminal amino acids, including the N-terminus myristylation signal eliminated CD4 and MHC-1 down-regulation, while maintaining most CTL, T-helper and B-cell epitopes (see Peng B, Robert-Guroff M (2001). Deletion of N-terminal myristoylation site of HIV Nef abrogates both MHC-1 and CD4 down-regulation. Immunol Lett. 78(3):195-200). Other groups have demonstrated that mutation of the Nef amino-terminal glycine (Gly2) into alanine prevents myristylation (see Liang, X. et al. (2002). Development of HIV-1 Nef vaccine components: immunogenicity study of Nef mutants lacking myristylation and dileucine motif in mice. Vaccine 20: 3413-3421, and Kaminchik, J. et al. (1991). Genetic Characterization of Human Immunodeficiency Virus Type 1 nef Gene Products Translated in vitro and Expressed in Mammalian Cells. J. of Virol. 65(2): 583-588).

Since the amino-terminal motif MG*XXX* of the Clade A Nef is embedded within the GRIN fusion protein, there is no nascent methionine to be cleaved by the methionine amino peptidase during translation. Thus, no newly generated amino-terminal group of glycine occurs and NMT is unable to execute myristylation. In conclusion, the inability of Nef in GRIN to undergo myristylation abrogates the biological function of Nef.

### Example 8: Codon optimization for GRIN (GagPolNef) and Env

The codon usage for each of GRIN and Env was adapted to the codon bias of human genes. The nucleotide and amino acid sequence of the codon optimized GRIN sequence is provided in FIGS. 16A-16J. The nucleotide and amino acid sequence of the codon optimized Env sequence is provided in FIGS. 17A-17D.

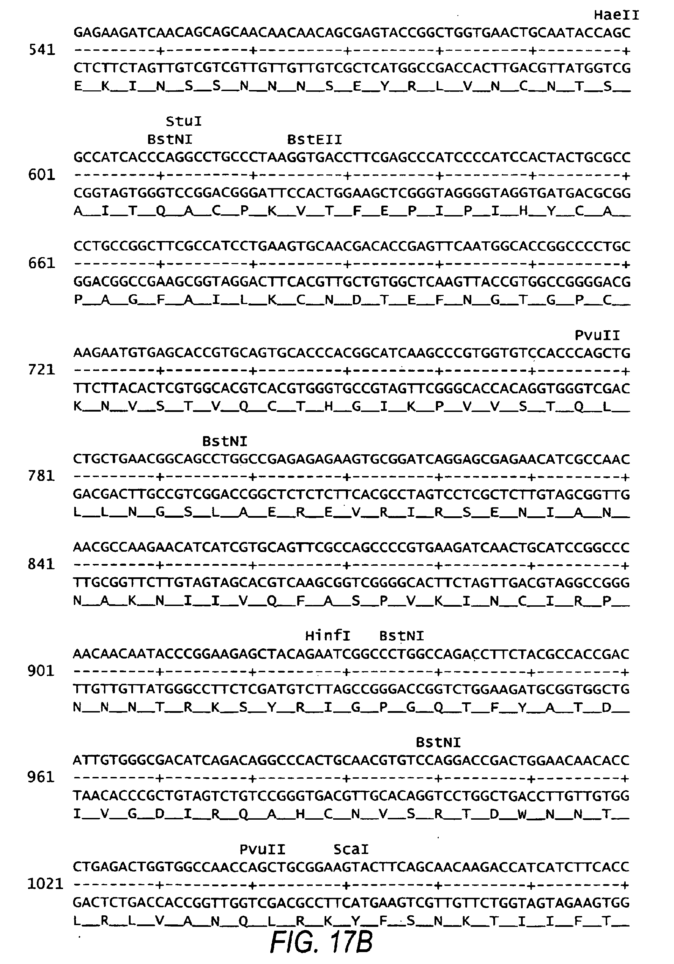

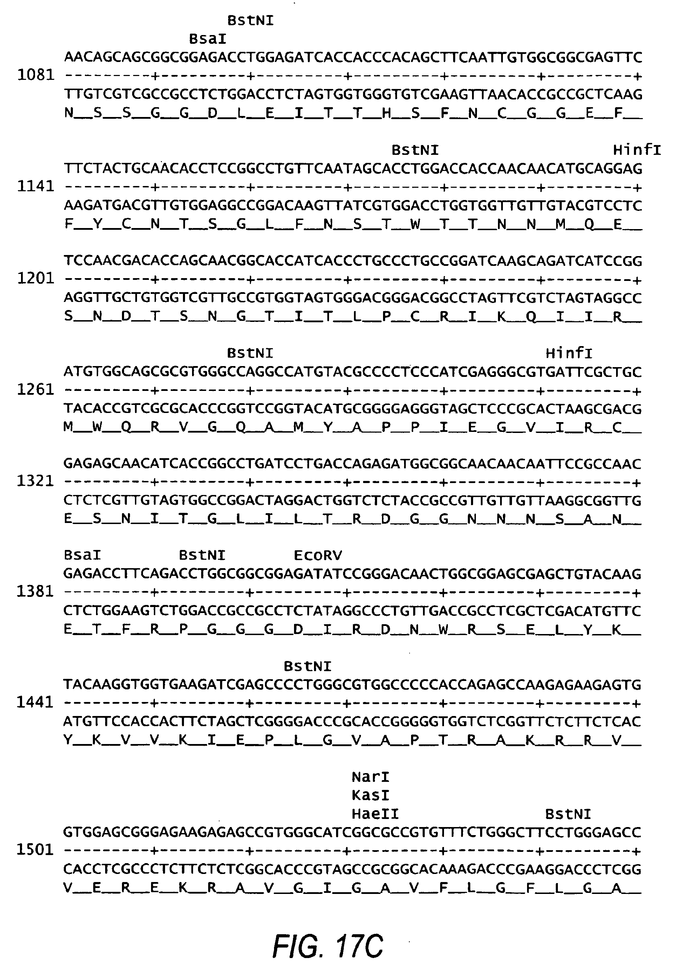

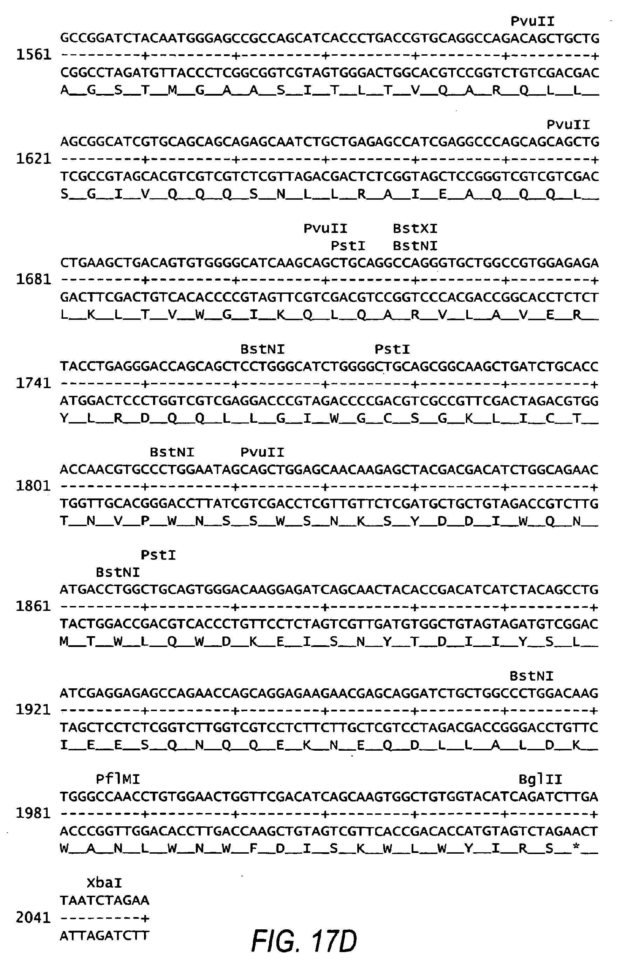

Regions of very high (greater than 80%) or very low (less than 30%) GC content were avoided where possible. During the optimization process the following cis-acting motifs were avoided: internal TATA boxes, chi-sites, ribosomal entry sites, AT-rich or GC-rich sequence stretches, ARE, INS, or CRS sequence elements, repeat sequences, RNA secondary structures, cryptic splice donor and acceptor sites, branch points, and HindIII, NcoI, BglII and BcII restriction sites except as indicated in the sequences provided in FIGS. 16 and 17. Also, a Kozak sequence was introduced upstream of the starting ATG for each of GRIN and Env to increase translation initiation, and two stop codons were added to each of GRIN and Env to ensure efficient termination. Restrictions sites to facilitate subcloning were also added, as indicated in FIGS. 16 and 17.

### Example 9: Non-human primate study

A non-human primate (Chinese rhesus macaques) study was conducted with the primary objective to assess the immunogenicity of GRIN and ENV in a human adenovirus type 35 (Ad35) vector delivery system. Animals were given increasing doses of Ad35-GRIN/ENV (10⁹, 10¹⁰ and 10¹¹ virus particles [vp]; intramuscular route) and received two immunizations at month 0 and month 6 (with 8 animals per group for the first immunization and 4 animals per group for the second immunization). At various timepoints (from week 0 through to week 50), animals were bled and immunogenicity measured by ELISpot for IFN-gamma (see FIGS. 23A and 23B for the 10¹⁰ and 10¹¹ vp dosages, respectively).

A dose response was observed (data for 10⁹ vp not shown), both in ELISPot intensity and frequency of responders following the prime (data not shown). Responses were seen to all vaccine antigen components of GRIN/ENV and IFNγ ELISPOT responses were boosted after the second immunization at month 6.

The disclosure is further described by the following numbered paragraphs:
1. A consensus nucleotide sequence for HIV-1 Clade A antigens, wherein the sequence comprises nucleotide sequences encoding HIV-1 Clade A Gag, Pol (RT and Int), and Nef ("GRIN), HIV-1 Clade A Gag, RT and Nef ("GRN") or HIV-1 Clade A Env.
2. A consensus nucleotide sequence according to paragraph 1 wherein the encoded Gag protein has the amino acid sequence of FIG. 1.
3. A consensus nucleotide sequence according to paragraph 1 wherein the encoded Pol protein has the amino acid sequence of FIG. 3.
4. A consensus nucleotide sequence according to paragraph 1 wherein the encoded Env protein has the amino acid sequence of FIG. 5.
5. A consensus nucleotide sequence according to paragraph 1 wherein the encoded Nef protein has the amino acid sequence of FIG. 7.
6. A method of identifying an HIV-1 Clade A antigen from a circulating strain or field isolate of HIV-1 that has an amino acid sequence that is similar to the consensus amino acid sequence for that HIV-1 Clade A antigen, comprising comparing the amino acid sequences of antigens from circulating strains or field isolates of HIV-1 to the consensus amino acid sequence for that protein, and selecting an antigen from the circulating strains or field isolates of HIV-1 that has a small protein distance from the consensus sequence.
7. An HIV-1 Clade A antigen identified using the method of paragraph 6.
8. An method of producing a transgenic HIV-1 Clade A antigen comprising selecting an HIV-1 Clade A antigen using the method of paragraph 6 and mutating the nucleotide sequence that encodes the antigen wherein the mutation abrogates the function of that antigen.
9. A method of generating an immune response against HIV-1 comprising administering to a subject a composition comprising a nucleotide sequence or antigen according to any of the previous paragraphs.

## Claims

1. A polynucleotide encoding an HIV-1 Clade A fusion protein, wherein the fusion protein comprises HIV-1 Clade A Gag, Pol and Nef and wherein the fusion protein comprises the amino acid sequence of Figure 16.

2. An immunogenic composition comprising the polynucleotide of claim 1.

3. The polynucleotide of claim 1 for use in a method of generating an immune response against HIV-1.

## Patentansprüche

1. Polynukleotid kodierend für ein HIV-1 Klade A Fusionsprotein, wobei das Fusionsprotein HIV-1 Klade A Gag, Pol und Nef umfasst und, wobei das Fusionsprotein die Aminosäuresequenz aus Figur 16 umfasst.

2. Immunogene Zusammensetzung umfassend das Polynukleotid nach Anspruch 1.

3. Polynukleotid nach Anspruch 1 zur Verwendung in einem Verfahren zur Erzeugung einer Immunantwort gegen HIV-1.

## Revendications

1. Polynucléotide codant une protéine de fusion du VIH-1 de clade A, dans lequel la protéine de fusion comprend Gag, Pol et Nef du VIH-1 de clade A et dans lequel la protéine de fusion comprend la séquence d'acide aminé de la figure 16.

2. Composition immunogène comprenant le polynucléotide selon la revendication 1.

3. Polynucléotide selon la revendication 1 pour utilisation dans un procédé pour générer une réponse immune contre le VIH-1.
